# EUROPÄISCHE PATENTSCHRIFT

(11) **EP 2 234 949 B1**
(45) Veröffentlichungstag und Bekanntmachung des Hinweises auf die Patenterteilung: **20.02.2013**
(21) Anmeldenummer: 08863003.3
(22) Anmeldetag: 17.12.2008
(51) Int. Cl.: C07C 37/07, C07C 37/20, C07C 37/62, C07C 39/15, C07C 39/367, C07C 45/74, C07C 49/613, C07C 49/653

(54) **VERFAHREN ZUR HERSTELLUNG VON 4,4'-[1-(TRIFLUORMETHYL)ALKYLIDEN]-BIS-(2,6-DIPHENYLPHENOLEN)**
METHOD FOR THE PRODUCTION OF 4,4'-[1-(TRIFLUOROMETHYL)ALKYLIDENE]-BIS-(2,6-DIPHENYLPHENOLS)
PROCÉDÉ DE PRODUCTION DE 4,4'-[1-(TRIFLUOROMÉTHYL)ALKYLIDÈNE]-BIS-(2,6-DIPHÉNYLPHÉNOLES)

(30) Priorität: 17.12.2007 EP 07123368
(43) Veröffentlichungstag der Anmeldung: 06.10.2010
(73) Patentinhaber: BASF SE, 67056 Ludwigshafen (DE)
(72) Erfinder: GRALLA, Gabriele, 68161 Mannheim (DE); HEYDRICH, Gunnar, 67117 Limburgerhof (DE); EBEL, Klaus, 68623 Lampertheim (DE); KRAUSE, Wolfgang, 68782 Brühl-Rohrhof (DE)
(86) Internationale Anmeldenummer: PCT/EP2008/067686
(87) Internationale Veröffentlichungsnummer: WO 2009/077549

(56) Entgegenhaltungen:
- DE-A1- 1 643 402
- DE-A1- 1 643 403
- DE-A1- 2 211 721
- US-A- 3 739 035
- B. H. BIBO, H. L. LOUWERSE: "Analysis of 2,6-Substituted Cycl6hexanones and Phenols by Gas-Chromatography Combined with Thin-Layer Chromatography" FRESENIUS' ZEITSCHRIFT FUER ANALYTISCHE CHEMIE, Bd. 236, 1968, Seiten 208-215, XP002528822

## Beschreibung

Die vorliegende Erfindung betrifft ein Verfahren zur Dehydrierung von durch Selbstkondensation von Cyclohexanon erhältlichen tricyclischen Kondensationsprodukten in Gegenwart eines geträgerten Übergangsmetallkatalysators in kondensierter Phase unter Bildung von 2,6-Diphenylphenol sowie ein Verfahren zur Herstellung von 4,4'-[1-(Trifluormethyl)alkyliden]-bis-(2,6-diphenylphenolen), insbesondere zur Herstellung von 4,4'-[1-(Trifluormethyl)ethyliden]-bis-(2,6-diphenylphenol) umfassend die Selbstkondensation von Cyclohexanon in Gegenwart eines basischen Katalysators unter Bildung von tricyclischen Kondensationsprodukten, Dehydrierung der erhaltenen tricyclischen Kondensationsprodukte In Gegenwart eines geträgerten Übergangsmetallkatalysators in kondensierter Phase unter Bildung von 2,6-Diphenylphenol und Umsetzung des 2,6-Diphenylphenols mit einem Trifluormethylketon. Die Erfindung betrifft darüber hinaus ein verbessertes Verfahren zur Herstellung von 2,6-Diphenylphenol durch Aldol-Selbstkondensation von Cyclohexanon und anschließender Dehydrierung.

4,4'-[1-(Trifluormethyl)ethyliden]-bis-(2,6-diphenylphenol) und 4,4'-[1,1-Bis-(trifluormethyl)methyliden]-bis-(2,6-diphenylphenol) sind aus der US 3,739,035 bekannt und werden als wertvolle Ausgangsstoffe zur Herstellung von Polycarbonaten oder Polyestern beschrieben. Die Herstellung erfolgt durch Umsetzung von 2,6-Diphenylphenol mit jeweils großen Überschüssen von gasförmigem Hexafluoraceton oder 1,1,1-Trifluoraceton in Methansulfonsäure.

Die genannten Verbindungen stellen auch wichtige Ausgangsstoffe zur Herstellung von Bis(diarylphenoxy)-Aluminiumverbindungen dar, wie sie in der WO 2006/092433 beschrieben sind.

Die DE 1 643 402 betrifft ein Verfahren zur Herstellung von 2,6-Diphenylphenol durch Selbstkondensation von Cyclohexanon unter Bildung tricyclischer Kondensationsprodukte und anschließender Dehydrierung derselben. Dabei wird die Selbstkondensation von Cyclohexanon unter Lösemittel-freien Bedingungen bei Temperaturen von bis zu 200°C in Gegenwart einer starken Base, bevorzugt wässrigen Lösungen von Natrium - oder Kaliumhydroxid, als Katalysator durchgeführt. Die im zweiten Schritt durchzuführende Dehydrierung der im Gemisch mit bicyclischen Kondensationsprodukten erhaltenen tricyclischen Kondensationsprodukte wird in Gegenwart eines Dehydrierkatalysators bei einer Temperatur von bis zu 350°C, bevorzugt 300 bis 350°C durchgeführt. Als geeignete Dehydrierkatalysatoren werden geträgerte Platin-, Palladium-, Nickel-, Ruthenium- und Rhodlumkatalysatoren beschrieben.

Die DE 1 643 403 offenbart ein Verfahren zur Kristallisation von 2,6-Diphenylphenol aus einer Mischung, die 2,6-Diphenylphenol neben mindestens einem weiteren Phenol, das in 2- bzw. 6-Position anstelle eines Phenylringes einen aliphatischen sechsgliedrigen Ring aufweist, enthält. Die Gemische werden dazu in einer Mischung von 75 bis 99 Gew.-% eines aliphatischen mit 1 bis 25 Gew.-% eines aromatischen Lösungsmittels gelöst und die Temperatur der Lösung auf einen Punkt unterhalb der Kristallisationstemperatur des 2,6-Diphenylphenols abgesenkt.

Die DE 2 211 721 betrifft ein Verfahren zur Herstellung von ortho-Phenylphenol, das dadurch gekennzeichnet ist, dass das Produkt der bimolekularen Dehydratation-Kondensation von Cyclohexanon zu einem Bett eines auf einem inaktiven Träger getragenen Katalysators zugeführt und das Kondensat der Dehydrierung bei 230 bis 520°C in Anwesenheit eines Inertgases unterworfen wird. Die Schrift offenbart auch Katalysatoren, die zur Durchführung des Verfahrens geeignet sind und die eines oder mehrere der Elemente Palladium, Platin, Iridium und Rhodium enthalten und zusätzlich ein Alkali enthalten können.

In Fresenius' Zeitschrift für analytische Chemie, Bd. 236, 1968, Seiten 208-215 wird die Autokondensation von Cyclohexanon und die Behandlung des so erhaltenen Gemisches von Cis-2,6-Di(cyclohexen-1-yl)cyclohexanon und dessen (Stereo-)Isomeren mit einem Palladium-Katalysator (Pd auf Kohle) beschrieben, wobei ein kompliziertes Gemisch vieler in struktureller Hinsicht ähnlicher 2,6-disubstituierter Cyclohexanone und Phenole erhalten wird.

Ausgehend von diesem Stand der Technik bestand die der vorliegenden Erfindung zu Grunde liegende Aufgabe in der Bereitstellung eines Verfahrens, dass die Herstellung von 4,4'-[1-(Trifluormethyl)alkyliden]-bis-(2,6-diphenylphenolen) bzw. 2,6-Diphenylphenol auf besonders wirtschaftliche Weise, d.h. in möglichst hoher Ausbeute der gewünschten Verbindungen und mit möglichst geringer Bildung von unerwünschten, gegebenenfalls aufwändig abzutrennenden und zu entsorgenden oder erneut umzusetzenden Nebenprodukten in verfahrenstechnisch möglichst vorteilhafter Weise ermöglicht.

Die Aufgabe wurde erfindungsgemäß gelöst durch die Bereitstellung eines Verfahrens zur Dehydrierung von Verbindungen der Formel (IIa), (IIb) und/oder (IIc) 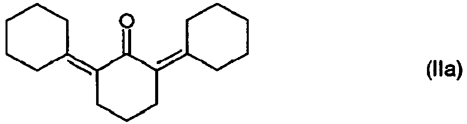 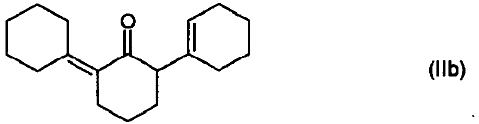 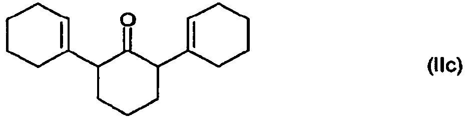 in Gegenwart eines geträgerten Übergangsmetallkatalysators in kondensierter Phase unter Bildung eines 2,6-Diphenylphenols der Formel (III) 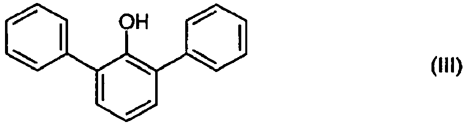 umfassenden Reaktionsgemisches, dadurch gekennzeichnet, dass man die Dehydrierung in Gegenwart eines auf Al₂O₃ geträgerten Pd-Katalysators und in Gegenwart von Carbonaten von Alkalimetallen durchführt.

Ein weiterer Gegenstand der Erfindung ist ein Verfahren zur Herstellung von 4,4'-[1-(Trifluormethyl)alkyliden]-bis-(2,6-diphenylphenolen) der Formel (I) 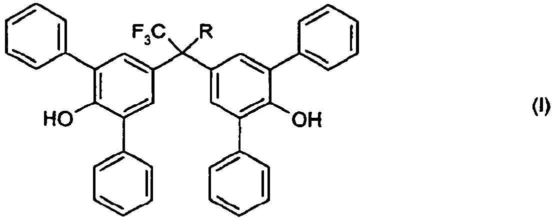 wobei der Rest
- R: unverzweigtes oder verzweigtes C₁- bis C₆-Alkyl oder C₁- bis C₆-Perfluoralkyl
bedeutet,
umfassend die Verfahrensschritte
a) Umsetzung von Cyclohexanon in Gegenwart eines basischen Katalysators unter Bildung eines Reaktionsgemisches umfassend die tricyclischen Kondensationsprodukte der Formel (IIa), (IIb) und/oder (IIc) 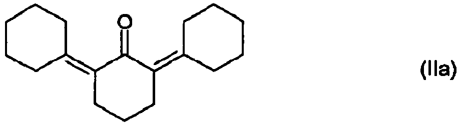 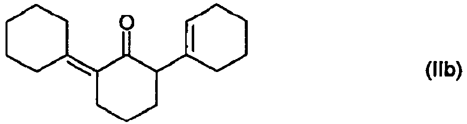 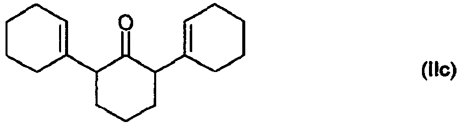 und unter Bildung von Wasser,
b) Abtrennung eines Gemisches der tricyclischen Kondensationsprodukte umfassend die Verbindungen der Formeln (IIa), (IIb) und/oder (IIc) von dem in Schritt a) gebildeten Reaktionsgemisch,
c) Dehydrierung der in Schritt b) erhaltenen tricyclischen Kondensationsprodukte umfassend die Verbindungen der Formeln (IIa), (IIb) und/oder (IIc) in Gegenwart eines geträgerten Übergangsmetallkatalysators in kondensierter Phase unter Bildung eines 2,6-Diphenylphenol der Formel (III) 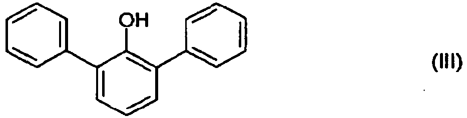 umfassenden Reaktionsgemisches gemäß dem eingangs beschriebenen, erfindungsgemäßen Verfahren zur Dehydrierung,
d) Abtrennung von 2,6-Diphenylphenol der Formel (III) von dem in Schritt c) gebildeten Reaktionsgemisch und
e) Umsetzung des In Schritt d) erhaltenen 2,6-Diphenylphenols der Formel (III) mit einem Trifluormethylketon der Formel (IV) 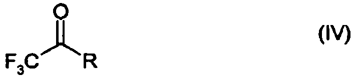 wobei der Rest R die gleiche Bedeutung wie in Formel (I) aufweist, in Gegenwart einer starken organischen Säure unter Bildung des 4,4'-[1-(Trifluormethyl)alkyliden]-bis-(2,6-diphenylphenols) der Formel (I).

Das weitere erfindungsgemäße Verfahren eignet sich zur Herstellung von 4,4'-[1-(Trifluormethyl)alkyliden]-bis-(2,6-diphenylphenolen) der Formel (I) 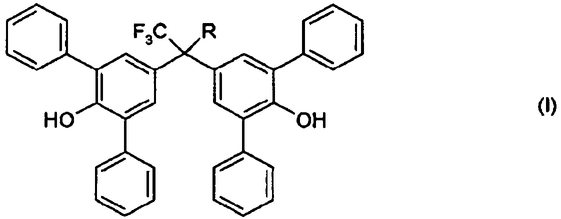 wobei der Rest R unverzweigtes oder verzweigtes C₁- bis C₆-Alkyl oder C₁- bis C₆-Perfluoralkyl bedeutet. Unter dem Begriff verzweigtes oder unverzweigtes C₁- bis C₆-Alkyl sind dabei verzweigte oder unverzweigte Alkylreste mit 1 bis 6 Kohlenstoffatomen wie beispielsweise Methyl, Ethyl, Propyl, iso-Propyl, Butyl, sec-Butyl, tert-Butyl, Pentyl oder Hexyl zu verstehen. Bevorzugte C₁- bis C₆-Alkylreste sind Methyl, Ethyl, iso-Propyl, besonders bevorzugt Methyl. Unter dem Begriff verzweigtes oder unverzweigtes C₁- bis C₆-Perfluoralkyl sind verzweigte oder unverzweigte Perfluoralkylreste, d.h. Alkylreste, bei denen alle Wasserstoffatome durch Fluoratome ersetzt sind, mit 1 bis 6 Kohlenstoffatomen zu verstehen wie beispielsweise Trifluormethyl, Pentafluorethyl, Heptafluorpropyl, Heptafluor-iso-Propyl, Nonafluorbutyl. Bevorzugte C₁- bis C₆-Perfluoralkylreste sind Trifluormethyl, Pentafluorethyl, Heptafluor-iso-Propyl, besonders bevorzugt Trifluormethyl. Mögliche besonders bevorzugte Verfahrensprodukte sind demnach 4,4'-[1-(Trifluormethyl)ethyliden]-bis-(2,6-diphenylphenol) der Formel (Ia) 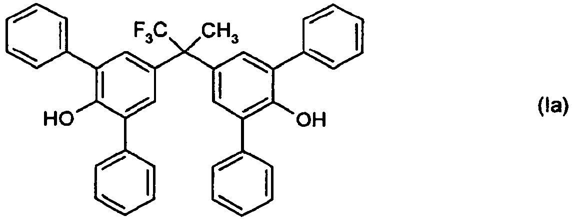 oder auch 4,4'[1,1-(Bis-Trifluormethyl)methyliden]-bis-(2,6-diphenylphenol) der Formel (Ib)

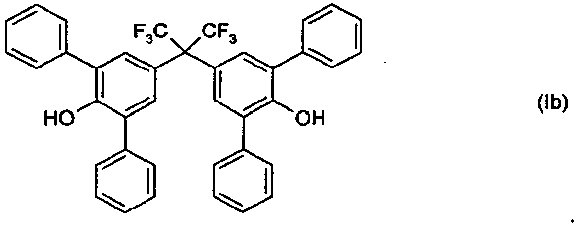

Ein erfindungsgemäß insbesondere bevorzugtes Verfahrensprodukt ist 4,4'-[1-(Trifluormethyl)ethyliden]-bis-(2,6-diphenylphenol) der Formel (Ia).

Das weitere erfindungsgemäße Verfahren umfasst die Verfahrenschritte a) bis e). Gemäß Verfahrenschritt a) des erfindungsgemäßen Verfahrens führt man eine Umsetzung von Cyclohexanon in Gegenwart eines basischen Katalysators unter Bildung eines Reaktionsgemisches umfassend die tricyclischen Kondensationsprodukte der Formel (IIa), (IIb) und/oder (IIc) 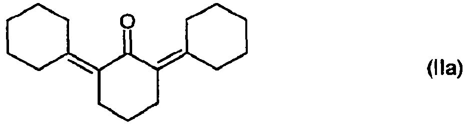 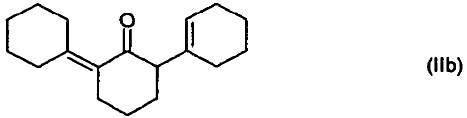 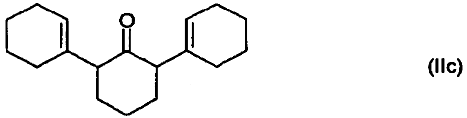 und unter Bildung von Wasser durch. Als Ausgangsstoff zur Durchführung des erfindungsgemäßen Verfahrens dient demnach Cyclohexanon. Dieses kann das in handelsüblicher Reinheit, d.h. ohne besondere Anforderungen an Reinheit, Herstellverfahren oder Beschaffenheit eingesetzt werden, üblicherweise in einer Reinheit von etwa 95 Gew.-% oder darüber, bevorzugt von 99 Gew.-% oder darüber.

Bei der Umsetzung von Cyclohexanon gemäß Schritt a) des erfindungsgemäßen Verfahrens handelt es sich um eine intermolekulare Selbstkondensation von 3 Molekülen Cyclohexanon im Sinne einer Aldolkondensation (Aldoladdition mit anschließender Wasserabspaltung), wie sie dem Fachmann an sich bekannt ist. Dabei entstehen Produktgemische tricyclischer Cyclohexanone, die die vorstehend abgebildeten Verbindungen der Formeln (IIa), (IIb) und/oder (IIc) umfassen. Die genannten Gemische können eine, zwei oder alle drei der genannten Verbindungen (IIa), (IIb) und (IIc) und gegebenenfalls noch weitere Isomere der genannten Verbindungen enthalten, beispielsweise solche, bei denen eine ethylenische Doppelbindung im Cyclohexanonring des Moleküls lokalisiert ist. Üblicherweise liegen alle drei tricyclischen Ketone der Formeln (IIa), (IIb) und (IIc) in den genannten Produktgemischen vor.

Als Nebenprodukte der gemäß Schritt a) des erfindungsgemäßen Verfahrens durchzuführenden Selbstkondensation von Cyclohexanon treten unerwünschterweise in der Regel auch bicyclische Cyclohexanone, speziell solche der Formeln (Va) und/oder (Vb) 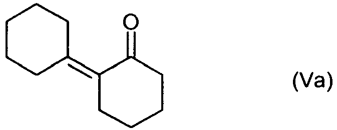 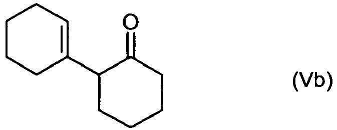 auf. Diese können jedoch, wie nachfolgend unter Schritt b) des erfindungsgemäßen Verfahrens beschrieben, von den tricyclischen Reaktionsprodukten der Formeln (IIa), (IIb) und/oder (IIc) abgetrennt werden, vorzugsweise durch destillative Verfahren, und gewünschtenfalls wieder in die Umsetzung gemäß Verfahrensschritt a) zurückgeführt werden.

Die Umsetzung gemäß Verfahrensschritt a) wird in Gegenwart eines basischen Katalysators, vorzugsweise in Gegenwart eines anorganischen, insbesondere stark basischen Katalysators durchgeführt. Als basische bzw. stark basische, insbesondere anorganische Katalysatoren bzw. Basen seien solche genannt, die in der Lage sind, Cyclohexanon zumindest teilweise durch Deprotonierung in das entsprechende Enolat-Anion zu überführen. Die Umsetzung gemäß Verfahrensschritt a) wird bevorzugt in Gegenwart einer starken Base durchgeführt, besonders bevorzugt einer solchen, die einen pKb-Wert von kleiner als 4 aufweist. Als bevorzugte starke Basen seien hierfür die Hydroxide, Alkoholate, Hydride, Amide oder Carbonate von Alkali- oder Erdalkalimetallen genannt wie beispielsweise Lithium-, Natrium-, Kalium-, Calcium- und Bariumhydroxid, Natriumethanolat, Natriummethanolat, Kalium-tert-Butylat, Natrium- und Kaliumhydrid, Lithiumdiisopropylamid sowie Lithium-, Natrium-, Kalium-, Calcium- und Bariumcarbonat. Insbesondere bevorzugte starke Basen sind die Hydroxide- und Carbonate der Alkali- oder Erdalkalimetalle, ganz besonders bevorzugt die Hydroxide der Alkalimetalle. Die genannten Verbindungen können in reiner Form oder in Form von Gemischen untereinander oder in Form von Gemischen mit anderen Basen eingesetzt werden. Sie können in fester oder gelöster Form, bevorzugt in Form von wässrigen Lösungen eingesetzt werden.

Die Menge an im Rahmen von Verfahrensschritt a) einzusetzenden basischen Katalysators ist nicht kritisch und kann über einen breiten Bereich variiert werden. Unter Berücksichtigung des ökomomischen Aspekts ist es jedoch vorteilhaft, den Katalysator in möglichst geringer Menge, bevorzugt in einer Menge von bis zu 20 mol-%, besonders bevorzugt von bis zu 10 mol-% und ganz besonders bevorzugt von bis zu 5 mol-%, jeweils bezogen auf die Basenäquivalente und die Menge an eingesetztem Cyclohexanon, einzusetzen.

Bevorzugt setzt man im Rahmen von Verfahrensschritt a) des erfindungsgemäßen Verfahrens als basischen Katalysator eine wässrige Lösung eines Alkali- oder Erdalkalihydroxids, insbesondere bevorzugt eine wässrige Lösung von Natriumhydroxid ein. Setzt man die gewählte Base in Form einer Lösung, bevorzugt in Form einer wässrigen Lösung ein, beträgt der bevorzugte Konzentrationsbereich dieser Lösungen etwa 5 bis etwa 50 Gew.-% (bezogen auf die fertige Lösung), besonders bevorzugt etwa 25 bis etwa 50 Gew.-%.

Die gemäß Verfahrensschritt a) durchzuführende Selbstkondensation von Cyclohexanon kann in einem breiten Temperaturbereich durchgeführt werden, üblicherweise bei Temperaturen von etwa 70°C bis zu etwa 200°C. Ein bevorzugter Temperaturbereich zur Durchführung von Verfahrensschritt a) des erfindungsgemäßen Verfahrens ist der Bereich von 90 bis 180°C.

Im Verlauf der Selbstkondensation des eingesetzten Cyclohexanons, d.h. mit fortschreitendem Umsatz bilden sich als primäre Kondensationsprodukte der Reaktion jeweils zweier Moleküle Cyclohexanon zunächst die dimeren, bicyclischen Kondensationsprodukte der Formeln (Va) und (Vb), die einen höheren Siedepunkt als Cyclohexanon selbst aufweisen und die zur Bildung der gewünschten tricyclischen Kondensationsprodukte der Formeln (IIa), (IIb) und/oder (IIc) mit einem weiteren Molekül Cyclohexanon reagieren müssen.

Eine erfindungsgemäß besonders bevorzugte Ausführungsform des erfindungsgemä-βen Verfahrens ist dadurch gekennzeichnet, dass man die Umsetzung gemäß Verfahrensschritt a) in Gegenwart eines mit Wasser ein Azeotrop bildenden (von Cyclohexanon verschiedenen) Lösungsmittels oder Lösungsmittelgemisches, durchführt. Bevorzugte "mit Wasser ein Azeotrop bildende Lösungsmittel" sind dabei unter den Reaktionsbedingungen inerte, vorzugsweise organische Lösungsmittel, die einen Siedepunkt bei Atmosphärendruck von etwa 100°C bis etwa 200°C, bevorzugt im Bereich von 100°C bis 150°C, besonders bevorzugt im Bereich von 110°C bis 140°C und ganz besonders bevorzugt im Bereich von 130°C bis 140°C aufweisen und von Cyclohexanon verschiedenen sind. Besonders bevorzugt sind solche organischen Lösungsmittel, die mit Wasser ein Azeotrop bilden, das einen niedrigeren Siedepunkt als Cyclohexanon, d.h. niedriger als 155°C, sowie einen niedrigeren Siedepunkt als das jeweilige Lösungsmittel selbst (tiefsiedendes Azeotrop) aufweisen. Insbesondere bevorzugt sind darunter solche Lösungsmittel oder Lösungsmittelgemische deren azeotroper Siedepunkt unterhalb des azeotropen Siedepunkts des Cyclohexanons von 95°C liegt. Zur Gewährleistung einer ausreichenden Reaktionsgeschwindigkeit ist es dabei vorteilhaft, wenn der azeotrope Siedepunkt des gewählten Lösungsmittels oder Lösungsmittelgemisches möglichst hoch, bevorzugt bei 70°C oder darüber, besonders bevorzugt bei 80°C oder darüber liegt. Erfindungsgemäß im Rahmen von Verfahrensschritt a) des erfindungsgemäßen Verfahrens besonders bevorzugt einsetzbare Lösungsmittel weisen demnach einen azeotropen Siedepunkt im Bereich von 70°C bis 95°C, bevorzugt von 80°C bis 95°C, insbesondere bevorzugt bis unterhalb 95°C auf wie beispielsweise Toluol, Xylol und Ethylbenzol oder Gemische derselben, bevorzugt Xylol. Das genannte "mit Wasser ein Azeotrop bildende Lösungsmittel" kann daher auch als Schleppmittel bezeichnet werden.

Die genannten Lösungsmittel können als solche oder in Form von Gemischen zweier oder mehrerer verschiedener Lösungsmittel eingesetzt werden. Bevorzugt setzt man im Rahmen von Verfahrensschritt a) des erfindungsgemäßen Verfahrens nur ein Lösungsmittel ein, bevorzugt ein solches, das mit Wasser ein wie vorstehend beschriebenes Azeotrop bildet.

Im Rahmen einer wiederum bevorzugten Ausführungsform führt man das erfindungsgemäße Verfahren so durch, dass man das in Verfahrensschritt a) durch Aldol-Selbstkondensation von Cyclohexanon gebildete Wasser in Form eines Azeotrops mit dem eingesetzten Lösungsmittel während der Umsetzung destillativ vom Reaktionsgemisch abtrennt. Die Abtrennung des im Rahmen der Aldol-Selbstkondensation von Cyclohexanon gebildeten Reaktionswassers sowie gegebenenfalls des in Form einer wässrigen Lösung des basischen Katalysators zugesetzten Wassers kann durch dem Fachmann an sich bekannte Verfahren der Azeotropdestillation unter Einsatz von ebenfalls bekannten Vorrichtungen zur Abtrennung bzw. Auskreisung von Wasser aus einem Reaktionsgemisch, wie beispielsweise einem Wasserabscheider, erfolgen. Die Wasserabscheidung kann vollständig bzw. weitestgehend vollständig oder auch nur teilweise vorgenommen werden. Bevorzugt trennt man jedoch die stöchiometrisch zu erwartende Menge an zu bildendem Wasser (sowie die gegebenenfalls mit dem Katalysator zugesetzte Menge an Wasser) möglichst weitgehend ab, um die gewünschte Bildung der genannten tricyclischen Reaktionsprodukte zu unterstützen.

Die Menge an im Rahmen dieser bevorzugten Ausführungsform einzusetzendem, mit Wasser ein Azeotrop bildendem Lösungsmittel kann in einem breiten Bereich gewählt werden und kann von verschiedenen Faktoren abhängig sein, insbesondere von der Wahl des konkret eingesetzten Lösemittels bzw. Lösemittelgemisches sowie nach dem Verfahren, bzw. der Vorrichtung, die zum Abtrennen bzw. Auskreisen des Wassers eingesetzt wird. Üblicherweise setzt man das gewählte Lösungsmittel, unter Berücksichtigung des ökonomischen Aspektes, in einer auf die eingesetzte Menge an Cyclohexanon bezogenen Menge von 5 bis 100 Gew. %, bevorzugt 10 bis 60 Gew.-% und besonders bevorzugt von 15 bis 40 Gew.-% ein.

Man erhält auf diese Weise ein Reaktionsgemisch, das neben dem eingesetzten basischen Katalysator im Wesentlichen die gewünschten tricyclischen Ketone der Formeln (IIa), (IIb) und/oder (IIc) neben bicyclischen Kondensationsprodukten der Formel (Va) und/der (Vb) sowie nicht umgesetztem Cyclohexanon enthält. Das so erhaltene Reaktionsgemisch kann in dieser Form weiterverarbeitet oder zunächst aufgearbeitet werden, beispielsweise durch dem Fachmann geläufige extraktive Verfahren. Vorteilhafterweise führt man zunächst eine Neutralisation des basischen Katalysators durch Behandlung mit einer Säure durch.

Gemäß Verfahrensschritt b) des erfindungsgemäßen Verfahrens trennt man aus dem so erhaltenen und gegebenenfalls aufgearbeiteten und weitgehend neutralisierten Reaktionsgemisch die tricyclischen Kondensationsprodukte umfassend die Verbindungen der Formeln (IIa), (IIb) und/oder (IIc) von dem in Verfahrensschritt a) gebildeten Reaktionsgemisch ab. Die Abtrennung kann nach dem Fachmann geeignet erscheinenden Verfahren, beispielsweise durch Chromatographie oder Destillation vorgenommen werden. Bevorzugt führt man die Abtrennung der tricylischen Reaktionsprodukte der Formeln IIa, IIb und/oder IIc vom gemäß Verfahrensschritt a) erhaltenen Reaktionsgemisch, gegebenenfalls nach Neutralisation und extraktiver Aufarbeitung, gemäß Verfahrensschritt b) in Form einer Destillation durch.

Die destillative Abtrennung der tricyclischen Kondensationsprodukte kann dabei diskontinuierlich, halb- oder auch vollkontinuierlich durchgeführt werden. Vorzugsweise führt man eine diskontinuierliche oder halbkontinuierliche Destillation, besonders bevorzugt eine diskontinuierliche Destillation durch. An die Ausgestaltung der einzusetzenden Destillationskolonne sind dabei keine besonderen Anforderungen zu stellen. Es können in vorteilhafter Weise gepackte Kolonnen, z.B. mit geeigneten Gewebepackungen, (Blechpackungen oder regellosen Schüttungen von Füllkörpern) gepackte Kolonnen eingesetzt werden. Die Destillation wird vorteilhaft bei vermindertem Druck, bevorzugt bei einem Sumpfdruck von etwa 1 bis etwa 100 mbar, besonders bevorzugt von etwa 5 bis etwa 30 mbar abs. und bei einem Kopfdruck von etwa 1 bis etwa 100 mbar abs., besonders bevorzugt von etwa 5 bis etwa 20 mbar abs. durchgeführt. Dementsprechend beträgt die Sumpftemperatur vorteilhafterweise etwa 200 bis etwa 250°C, bevorzugt etwa 210 bis etwa 230°C und die Kopftemperatur etwa 190 bis etwa 220°C, bevorzugt etwa 200 bis etwa 210°C. Dabei erhält die tricyclischen Ketone der Formeln (IIa), (IIb) und/oder (IIc) als hochsiedendes Sumpfprodukt, von dem die niedriger siedenden Komponenten, insbesondere die bicyclischen Kondensationsprodukte der Formeln (Va) und/oder (Vb) als Kopfprodukt abdestilliert werden. Diese können gewünschtenfalls wieder der Aldol-Selbstkondensation des Cyclohexanons gemäß Verfahrensschritt a) des erfindungsgemäßen Verfahrens als Ausgangsstoff zugeführt werden.

Gemäß Verfahrensschritt c) des erfindungsgemäßen Verfahrens führt man eine Dehydrierung der gemäß Verfahrensschritt b) erhaltenen tricyclischen Kondensationsprodukte umfassend die Verbindungen der Formel (IIa), (IIb) und/oder (IIc) in Gegenwart eines geträgerten Übergangsmetallkatalysators in kondensierter Phase unter Bildung eines 2,6-Diphenylphenol der Formel (III) 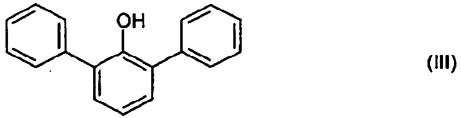 umfassenden Reaktionsgemisches durch, dadurch gekennzeichnet, dass man die Dehydrierung in Gegenwart eines auf Al₂O₃ geträgerten Pd-Katalysators und in Gegenwart von Carbonaten von Alkalimetallen durchführt.

Die Dehydrierung gemäß Verfahrensschritt c) des erfindungsgemäßen Verfahrens wird in kondensierter, d.h. flüssiger Phase durchgeführt. Dabei liegt das umzusetzende Gemisch umfassend die tricyclischen Ketone der Formeln (IIa), (IIb) und/oder (IIc) sowie das als Dehydrierungsprodukt erhaltene 2,6-Diphenylphenol der Formel (III) sowie gegebenenfalls noch anfallende teilweise dehydrierte Verbindungen wie beispielsweise 2-Cyclohexyl-6-phenylphenol weitgehend, d.h. zum überwiegenden Teil in flüssiger Form vor. Die Dehydrierung wird üblicherweise bei erhöhter Temperatur, bevorzugt bei Temperaturen im Bereich von etwa 200°Cbis zu etwa 300°C, d.h. bei Temperaturen unterhalb des Siedepunktes der genannten tricyclischen Ausgangsstoffe oder Produkte der Dehydrierung durchgeführt. Bevorzugt führt man die Dehydrierung bei einer Temperatur im Bereich von 240 bis 300°C, besonders bevorzugt im Bereich von 250 bis 300°C durch.

Al₂O₃ kann in Form von γ-Al₂O₃ (gamma-Al₂O₃) oder auch in Form von δ-Al₂O₃ (delta-Al₂O₃) oder in Form von θ-Al₂O₃ (theta-Al₂O₃) oder in Form von δ/θ-Al₂O₃ (delta/theta-Al₂O₃) oder in Form von α-Al₂O₃ (alpha-Al₂O₃), wie beispielsweise beschrieben in Hollemann Wiberg, Lehrbuch der Anorganischen Chemie, 102. Auflage, de Gruyter, 2007, Seite 1161, eingesetzt werden. Bevorzugt setzt man als Träger γ-Al₂O₃ (gamma-Al₂O₃) ein. Als im Rahmen der vorliegenden Erfindung besonders bevorzugter geträgerter Katalysator sei daher Pd auf γ-Al₂O₃ (gamma-Al₂O₃) genannt.

Palladium liegt im geträgerten Katalysator üblicherweise in einem Gewichtsanteil von etwa 0,1 bis etwa 20 Gew.-%, bevorzugt etwa 0,1 bis 10 Gew.-% (jeweils bezogen auf den fertigen Katalysator) vor. Es wird üblicherweise, je nach Art des eingesetzten Katalysators, in einer auf das Gewicht des zu dehydrierenden Gemisches tricyclischer Ketone bezogenen Menge von 1 bis 40 Gew.-%, bevorzugt 1 bis 35 Gew.-% eingesetzt.

Der erfindungemäß einzusetzende geträgerte Übergangsmetallkatalysator kann in einer breiten Vielfalt von dem Fachmann bekannten Formen eingesetzt werden, beispielsweise in Form von Kugeln, Strängen oder auch als Pulver.

Die Dehydrierung gemäß Verfahrensschritt c) wird zusätzlich zum eingesetzten geträgerten Übergangsmetallkatalysator in Gegenwart von Carbonaten von Alkalimetallen durchgeführt, beispielsweise in Gegenwart von Lithium-, Natrium- oder Kaliumcarbonat. Die genannten basischen Verbindungen können, je nach Art der eingesetzten Verbindung bzw. Verbindungen in eine Menge von üblicherweise 3 bis 20 Gew.-% bezogen auf den eingesetzten geträgerten Katalysator eingesetzt werden. Alternativ lassen sich auch mit wie vorstehend genannten Alkalicarbonaten vorbehandelte Träger bzw. Trägerkatalysatoren einsetzen.

Die Dehydrierung gemäß Verfahrensschritt c) erfolgt in der Regel rasch und ist bei den genannten Reaktionstemperaturen üblicherweise nach etwa 24 h, oft nach etwa 12 h oder darunter weitgehend abgeschlossen. Man erhält ein Reaktionsgemisch, das die vollständig dehydrierte Verbindung 2,6-Diphenylphenol der Formel (III), in der Regel neben nicht oder nur teilweise dehydrierten tricyclischen Ketonen, enthält.

Die Abtrennung der wie vorstehend beschriebenen heterogenen Dehydrierungskatalysatoren kann nach dem Fachmann geläufigen Verfahren, beispielsweise durch Filtration oder Zentrifugation, bevorzugt durch Filtration, durchgeführt werden. Bei Einsatz der wie vorstehend beschriebenen geträgerten Übergangsmetallkatalysatoren hat es sich gezeigt, dass die nach der Umsetzung gemäß Verfahrensschritt c) abgetrennten Katalysatoren in der Regel nach wie vor eine hohe Aktivität aufweisen. Sie können daher in vorteilhafter Weise wiederverwendet werden, vorzugsweise im Rahmen weiterer Umsetzungen gemäß Verfahrensschritt c). Im Rahmen einer bevorzugten Ausführungsform des erfindungsgemäßen Verfahrens trennt man daher den im Rahmen von Verfahrensschritt c) eingesetzten Katalysator nach erfolgter Umsetzung vom Reaktionsgemisch ab und setzt ihn im Rahmen einer oder mehrere weiterer Umsetzung gemäß Verfahrensschritt c) wieder ein.

Dabei kann der jeweils wieder gewonnene Katalysator im Prinzip so lange und sooft wieder eingesetzt werden, bis er nicht mehr die gewünschte Aktivität aufweist. Dies hängt in der Regel vom jeweils gewählten Katalysator, von den gewählten Ausgangsstoffen sowie von den Reaktionsbedingungen ab. Bei Einsatz von Palladium (Pd) auf einem Träger kann dieser üblicherweise bis zu etwa zehn mal oder öfter, zumindest jedoch bis zu fünf oder bis zu vier mal zurückgeführt, d.h. wieder eingesetzt werden, ohne dass dabei nennenswerte Aktivitäts- oder Selektivitätsverluste bei der Dehydrierungsreaktion auftreten.

Der wie vorstehend beschriebene, erfindungsgemäße Zusatz von Carbonaten von Alkalimetallen im Rahmen von Verfahrensschritt c) kann sich auch vorteilhaft auf die Aktivität, Haltbarkeit bzw. Wiederverwendbarkeit des jeweils eingesetzten geträgerten Übergangsmetallkatalysators auswirken. Der Zusatz von Alkalicarbonaten, bevorzugt von Natrium- und/oder Kaliumcarbonat und ganz besonders bevorzugt von Kaliumcarbonat (K₂CO₃) kann zu einer Aktivitätssteigerung und somit zu einer verbesserten Wiederverwendbarkeit des jeweils eingesetzten geträgerten Katalysators führen. Bei Umsetzungen mit geträgerten Palladiumkatalysatoren, speziell bei Umsetzungen mit dem erfindungsgemäß besonders bevorzugten Pd auf γ-Al₂O₃ (gamma-Al₂O₃) als Katalysator kann dieser Effekt zum Tragen kommen. Im Rahmen einer besonders bevorzugten Ausführungsform führt man demgemäß Schritt c) des erfindungsgemäßen Verfahrens in Gegenwart von Pd auf γ-Al₂O₃ (gamma-Al₂O₃) als geträgertem Übergangsmetallkatalysator und in Gegenwart eines Alkalicarbonats, bevorzugt in Gegenwart von Kaliumcarbonat durch.

Gemäß Verfahrensschritt d) des erfindungsgemäßen Verfahrens führt man eine Abtrennung von 2,6-Diphenylphenol der Formel (III) von dem in Verfahrensschritt c) gebildeten Reaktionsgemisch durch. Die Abtrennung gemäß Verfahrensschritt d) kann im Prinzip durch die gängigen Verfahren zur Stofftrennung wie beispielsweise Destillation, Chromatographie oder Kristallisation vorgenommen werden. Es hat sich als vorteilhaft erwiesen, 2,6-Diphenylphenol durch Kristallisation von den unerwünschten nicht oder nur teilweise dehydrierten tricyclischen Ketonen abzutrennen. Als geeignete Lösemittel haben sich dazu niedere Kohlenwasserstoffe mit bis zu 8 Kohlenstoffatomen wie beispielsweise Pentan, Hexan, Cyclohexan, Heptan, Octan, Toluol, Xylol, gegebenenfalls im Gemisch mit niederen aliphatischen Alkoholen mit 1 bis 4 Kohlenstoffatomen wie Methanol, Ethanol, Propanol, Isopropanol oder Butanol oder mit Ketonen, Ethern oder Estern mit bis zu 5 Kohlenstoffatomen wie beispielsweise Aceton, tert-Butylmethylether oder Essigester erwiesen. Es hat sich als besonders vorteilhaft erwiesen, die Abtrennung von 2,6-Diphenylphenol gemäß Verfahrensschritt d) in Form einer Kristallisation aus Heptan oder einem Heptan umfassenden Lösungsmittelgemisch durchzuführen. Als insbesondere geeignetes Lösungsmittel bzw. Lösungsmittelkombination hat sich reines Heptan oder ein Gemisch aus Heptan und iso-Propanol in einem Volumenverhältnis von etwa 20 zu 1 bis etwa 30 zu 1 erwiesen. Unter dem Begriff Heptan sind dabei sowohl n-Heptan als auch Isomere desselben wie beispielsweise 2-Methylhexan, 3-Methylhexan, 2,2-Dimethylpentan, 2,3-Dimethylpentan, 2,4-Dimethylpentan, 3,3-Dimethylpentan, 3-Ethylpentan, 2,2,3-Trimethylbutan oder Gemische derselben zu verstehen.

Das durch vorstehend beschriebene Kristallisation erhaltenen 2,6 Diphenylphenol der Formel (III) kann im Anschluss auf übliche Weise, bevorzugt durch Filtration oder Zentrifugation von der Mutterlage getrennt werden.

Auf diese Weise kann 2,6-Diphenylphenol der Formel (III) in reiner Form, d.h. in einer Reinheit von mindestens 98 Gew.-% oft von mindestens 99 Gew.-% erhalten werden. Dieses Material ist arm an unerwünschten, nicht oder nur teilweise dehydrierten tricyclischen Ketonen, die im Rahmen weiterer Verfahrensschritte bzw. Umsetzungen nur schwer abtrennbar wären und zu unerwünschten Produktgemischen und Nebenreaktionen führen würden.

Gemäß Verfahrensschritt e) des erfindungsgemäßen Verfahrens setzt man das in Verfahrensschritt d) erhaltene 2,6-Diphenylphenol der Formel (III) mit einem Trifluormethylketon der Formel (IV) 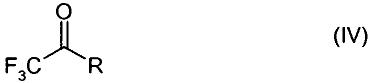 wobei der Rest R die gleiche Bedeutung wie in Formel (I) aufweist, in Gegenwart einer starken organischen Säure unter Bildung des 4,4'-[1-(Trifluormethyl)alkyliden]-bis-(2,6-diphenylphenols) der Formel (I) um.

Je nach gewünschter Zielverbindung setzt man demnach gemäß Verfahrensschritt e) das gemäß den Verfahrensschritte a) bis d) hergestellte 2,6-Diphenylphenol mit einem Trifluormethylketon der Formel (IV) um, wobei der Rest R wie vorstehend für die Verbindungen der Formel (I) beschriebenes C₁- bis C₆-Alkyl oder C₁- bis C₆-Perfluoralkyl bedeuten kann, um. Zur Herstellung der erfindungsgemäß besonders bevorzugten Verfahrensprodukte der Formeln (Ia) bzw. (Ib) setzt man das gemäß Verfahrensschritt d) erhaltene 2,6-Diphenylphenol demgemäß mit entweder 1,1,1-Trifluoraceton der Formel (IVa) 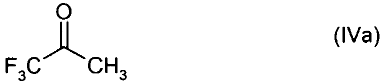 oder mit Hexafluoraceton der Formel (IVb) 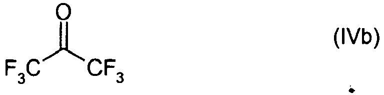 um. Beide Reagenzien können in handelsüblicher Form ohne besondere Anforderungen an Reinheit oder Herstellverfahren eingesetzt werden, wobei Hexafluoraceton der Formel (IVb) bevorzugt gasförmig in das Reaktionsgemisch eingeleitet wird.

Das gewählte Trifluormethylketon wird vorteilhafterweise der Stöchiometrie der Reaktion entsprechend, bevorzugt in leichtem Überschuss eingesetzt. Üblicherweise setzt man die Verbindungen 2,6-Diphenylphenol und das gewählte Trifluormethylketon der Formel (IV) in einem molaren Verhältnis von etwa 1 zu 1 bis etwa 2 zu 1, bevorzugt von etwa 2,0 zu 1,2 bis etwa 2,0 zu 1,1 ein.

Die Umsetzung gemäß Verfahrensschritt e) wird in Gegenwart einer starken organischen Säure durchgeführt, bevorzugt einer solchen mit einem pKa-Wert von bis zu 2, besonders bevorzugt mit einem pKa-Wert im Bereich von -1 bis 2, ganz besonders bevorzugt mit einem pKa-Wert im Bereich von 1 bis 2 durchgeführt. Als bevorzugte, im Rahmen von Verfahrensschritt e) einsetzbare organische Säure seien Sulfonsäuren, speziell Alkyl- oder Phenylsulfonsäuren genannt. Als bevorzugte Sulfonsäuren seien beispielsweise genannt: Methansulfonsäure, Trifluormethansulfonsäure, Benzolsulfonsäure, para-Toluolsulfonsäure, besonders bevorzugt Methansulfonsäure oder Trifluormethansulfonsäure und insbesondere bevorzugt Methansulfonsäure.

Die gewählte starke organische Säure, bevorzugt Methansulfonsäure oder Trifluormethansulfonsäure, besonders bevorzugt Methansulfonsäure, werden im Rahmen einer bevorzugten Ausführungsform in unverdünnter Form (100 %-ig) eingesetzt. Sie werden üblicherweise in deutlichem Überschuss zur Menge an umzusetzendem 2,6-Diphenylphenol eingesetzt. In der Regel wählt man, unter Berücksichtigung des ökonomischen Aspektes, ein gewichtsbezogenes Mengenverhältnis der gewählten Säure zum 2,6-Diphenylphenol von etwa 10 zu 1 bis etwa 30 zu 1, bevorzugt etwa 10 zu 1 bis etwa 20 zu 1.

Zur Durchführung der Reaktion gemäß Verfahrensschritt e) können die gewählten Reagenzien in beliebiger Reihenfolge miteinander in Kontakt gebracht werden, üblicherweise bei Temperaturen im Bereich von 0 bis 100°C. Bevorzugt führt man die Umsetzung gemäß Verfahrensschritt e) bei einer Temperatur im Bereich von 10 bis 60°C, besonders bevorzugt im Bereich von 20 bis 50°C durch. Die Umsetzung ist dann üblicherweise nach Reaktionszeiten von 10 bis zu 24 h weitgehend abgeschlossen.

Aus dem so zugänglichen Reaktionsgemisch kann durch übliche Abtrennverfahren, bevorzugt durch Filtration oder bevorzugt durch Extraktion, bevorzugt durch Extraktion mit Toluol, Xylol oder Ethylbenzol oder Gemischen derselben die in der Regel in fester Form anfallende Zielverbindung der Formel (I) isoliert werden. Im Rahmen einer bevorzugten Ausführungsform führt man das erfindungsgemäße Verfahren so durch, dass man die in Verfahrensschritt e) gebildete 4,4'-[1-(Trifluormethyl)alkyliden]-bis-(2,6-diphenylphenole) der Formel (I) durch Extraktion von dem erhaltenen Reaktionsgemisch abtrennt. Ein im Rahmen dieser Ausführungsform besonders bevorzugtes Extraktionsmittel ist Toluol. Auf diese Weise kann auch die eingesetzte organische Säure, bevorzugt die eingesetzte Methansulfonsäure oder Trifluormethansulfonsäure zurück gewonnen und gewünschtenfalls wieder eingesetzt werden, vorzugsweise im Rahmen einer weiteren Umsetzung gemäß Verfahrensschritt e) des erfindungsgemäßen Verfahrens. Das nach der im Rahmen dieser bevorzugten Ausführungsform durchzuführenden Extraktion in der wieder gewonnenen Methansulfonsäure gelöste jeweils eingesetzte Extraktionsmittel, bevorzugt Toluol, kann destillativ abgetrennt werden, um Nebenreaktionen mit 1,1,1-Trifluoraceton und Toluol zu vermeiden.

Das erfindungsgemäße Verfahren umfasst daher in einem weiteren optionalen Verfahrensschritt f) die Abtrennung der Zielverbindung der Formel (I) aus dem in Verfahrensschritt e) erhaltenen Reaktionsgemisch. Man erhält die gewünschte Zielverbindung üblicherweise in einer Reinheit von 95 Gew.-% oder darüber, bevorzugt in einer Reinheit von 97 Gew.-% oder darüber.

Das erfindungsgemäße Verfahren bietet demnach einen leistungsfähigen Zugang zu den gewünschten Zielverbindungen der Formel (I), insbesondere zu den Verbindungen der Formeln (Ia) oder (Ib), die im Rahmen der vorliegenden Erfindung als bevorzugte Zielverbindungen zu betrachten sind. Das erfindungsgemäße Verfahren ermöglicht die Herstellung der genannten Verbindungen in hoher Gesamtausbeute und hoher Reinheit.

Die vorliegende Erfindung betrifft in einem weiteren Aspekt ein Verfahren zur Herstellung von 2,6-Diphenylphenol der Formel (III) 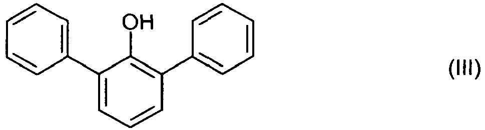 umfassend die Schritte
i) Umsetzung von Cyclohexanon in Gegenwart eines basischen Katalysators unter Bildung eines Reaktionsgemisches umfassend die tricyclischen Kondensationsprodukte der Formel (IIa), (IIb) und/oder (IIc) 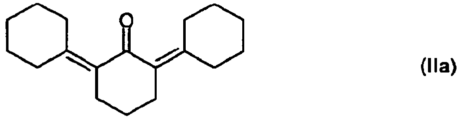 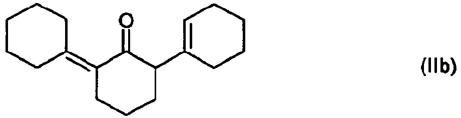 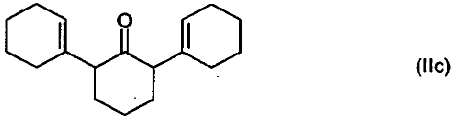 und unter Bildung von Wasser in Gegenwart eines mit Wasser ein Azeotrop bildenden, von Cyclohexanon verschiedenen Lösungsmittels oder Lösungsmittelgemisches, wobei man das gebildete Wasser in Form eines Azeotrops mit dem eingesetzten Lösungsmittel während der Umsetzung destillativ vom Reaktionsgemisch abtrennt,
ii) Abtrennung eines Gemisches der tricyclischen Kondensationsprodukte umfassend die Verbindungen der Formeln (IIa), (IIb) und/oder (IIc) von dem in Schritt i) gebildeten Reaktionsgemisch und
iii) Dehydrierung der in Schritt ii) erhaltenen tricyclischen Kondensationsprodukte umfassend die Verbindungen der Formeln (IIa), (IIb) und/oder (IIc) in Gegenwart eines geträgerten Übergangsmetallkatalysators in kondensierter Phase unter Bildung eines 2,6-Diphenylphenol der Formel (III) 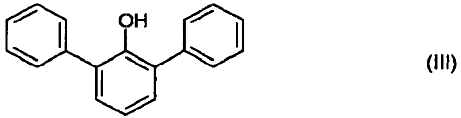 umfassenden Reaktionsgemisches gemäß dem eingangs beschriebenen, erfindungsgemäßen Verfahren zur Dehydrierung.

Dieser Aspekt der vorliegenden Erfindung betrifft demnach ein Verfahren zur Herstellung von 2,6-Diphenylphenol, das dem Verfahrensschritten a) bis c) des vorstehend beschriebenen Verfahrens entspricht, wobei man die Selbstkondensation von Cyclohexanon in Gegenwart eines basischen Katalysators gemäß Verfahrensschritt i) in Gegenwart eines mit Wasser ein Azeotrop bildenden (und von Cyclohexanon verschiedenen) Lösungsmittels oder Lösungsmittelgemisches durchführt und wobei man das gebildete Wasser in Form eines Azeotrops mit dem eingesetzten Lösungsmittel während der Umsetzung destillativ vom Reaktionsgemisch abtrennt.

Dem Begriff "mit Wasser ein Azeotrop bildendes Lösungsmittel" können dabei die gleichen generellen und bevorzugten Bedeutungen zukommen wie vorstehend unter Verfahrensschritt a) beschrieben. Demnach sind auch im Rahmen dieses Aspektes der vorliegenden Erfindung unter dem Begriff "mit Wasser ein Azeotrop bildende Lösungsmittel" unter den Reaktionsbedingungen inerte, vorzugsweise organische Lösungsmittel zu verstehen, die einen Siedepunkt bei Atmosphärendruck von etwa 100°C bis etwa 200°C, bevorzugt im Bereich von 100 bis 150°C, besonders bevorzugt im Bereich von 110 bis 140 °C und ganz besonders bevorzugt im Bereich von 130 bis 140°C aufweisen und von Cyclohexanon verschiedenen sind. Besonders bevorzugt sind solche organischen Lösungsmittel, die mit Wasser ein Azeotrop bilden, das einen niedrigeren Siedepunkt als Cyclohexanon, d.h. niedriger als 155°C, sowie einen niedrigeren Siedepunkt als das jeweilige Lösungsmittel selbst (tiefsiedendes Azeotrop) aufweisen. Insbesondere bevorzugt sind darunter solche Lösungsmittel oder Lösungsmittelgemische, deren azeotroper Siedepunkt unterhalb des azeotropen Siedepunkts des Cyclohexanons von 95°C liegt. Zur Gewährleistung einer ausreichenden Reaktionsgeschwindigkeit ist es dabei vorteilhaft, wenn der azeotrope Siedepunkt des gewählten Lösungsmittels oder Lösungsmittelgemisches möglichst hoch, bevorzugt bei 70°C oder darüber, besonders bevorzugt bei 80°C oder darüber liegt. Erfindungsgemäß im Rahmen von Verfahrensschritt a) des erfindungsgemäßen Verfahrens besonders bevorzugt einsetzbare Lösungsmittel weisen demnach einen azeotropen Siedepunkt im Bereich von 70°C bis 95°C, bevorzugt von 80°C bis 95°C, insbesondere bevorzugt bis unterhalb 95°C auf wie beispielsweise Toluol, Xylol und Ethylbenzol oder Gemische derselben, bevorzugt Xylol. Das genannte "mit Wasser ein Azeotrop bildende Lösungsmittel" kann daher auch als Schleppmittel bezeichnet werden.

Die genannten Lösungsmittel können als solche oder in Form von Gemischen zweier oder mehrerer verschiedener Lösungsmittel eingesetzt werden. Bevorzugt setzt man im Rahmen von Verfahrensschritt i) des erfindungsgemäßen Verfahrens nur ein Lösungsmittel ein, bevorzugt ein solches, das mit Wasser ein wie vorstehend beschriebenes Azeotrop bildet.

Im Rahmen dieses Aspekts der vorliegenden Erfindung führt man Verfahrensschritt i) des erfindungsgemäßen Verfahrens so durch, dass man das durch Aldol-Selbstkondensation von Cyclohexanon gebildete Wasser in Form eines Azeotrops mit dem eingesetzten Lösungsmittel während der Umsetzung destillativ vom Reaktionsgemisch abtrennt. Die Abtrennung des im Rahmen der Aldol-Selbstkondensation von Cyclohexanon gebildeten Reaktionswassers sowie gegebenenfalls des in Form einer wässrigen Lösung des basischen Katalysators zugesetzten Wassers kann durch dem Fachmann an sich bekannte Verfahren der Azeotropdestillation unter Einsatz von ebenfalls bekannten Vorrichtungen zur Abtrennung bzw. Auskreisung von Wasser aus einem Reaktionsgemisch, wie beispielsweise einem Wasserabscheider, erfolgen. Die Wasserabscheidung kann vollständig bzw. weitestgehend vollständig oder auch nur teilweise vorgenommen werden. Bevorzugt trennt man jedoch die stöchiometrisch zu erwartende Menge an zu bildendem Wasser (sowie die gegebenenfalls mit dem Katalysator zugesetzte Menge an Wasser) möglichst weitgehend ab, um die gewünschte Bildung der genannten tricyclischen Reaktionsprodukte zu unterstützen.

Die Menge an im Rahmen dieses Aspekts der vorliegenden Erfindung einzusetzendem, mit Wasser ein Azeotrop bildendem Lösungsmittel kann in einem breiten Bereich gewählt werden und kann von verschiedenen Faktoren abhängig sein, insbesondere von der Wahl des konkret eingesetzten Lösemittels bzw. Lösemittelgemisches sowie nach dem Verfahren, bzw. der Vorrichtung, die zum Abtrennen bzw. Auskreisen des Wassers eingesetzt wird. Üblicherweise setzt man das gewählte Lösungsmittel, unter Berücksichtigung des ökonomischen Aspektes, in einer auf die eingesetzte Menge an Cyclohexanon bezogenen Menge von 5 bis 100 Gew.%, bevorzugt 10 bis 60 Gew.-% und besonders bevorzugt von 15 bis 40 Gew.-% ein.

Bezüglich der im Rahmen von Verfahrensschritt i) einzusetzenden basischen Katalysatoren sowie bezüglich weiterer Merkmale dieses Verfahrensschritts sei vollumfänglich auf die vorstehende Beschreibung des Verfahrensschritts a) einschließlich aller bevorzugter Ausführungsformen und deren Kombinationen verwiesen.

Demgemäß wird auch die Umsetzung gemäß Verfahrensschritt i) in Gegenwart eines basischen Katalysators, vorzugsweise in Gegenwart eines anorganischen, insbesondere stark basischen Katalysators durchgeführt. Als basische bzw. stark basische, insbesondere anorganische Katalysatoren bzw. Basen seien solche genannt, die in der Lage sind, Cyclohexanon zumindest teilweise durch Deprotonierung in das entsprechende Enolat-Anion zu überführen. Die Umsetzung gemäß Verfahrensschritt i) wird bevorzugt in Gegenwart einer starken Base durchgeführt, besonders bevorzugt einer solchen, die einen pKb-Wert von kleiner als 4 aufweist. Als bevorzugte starke Basen seien hierfür die Hydroxide, Alkoholate, Hydride, Amide oder Carbonate von Alkali- oder Erdalkalimetallen genannt wie beispielsweise Lithium-, Natrium-, Kalium-, Calcium- und Bariumhydroxid, Natriumethanolat, Natriummethanolat, Kalium-tert-Butylat, Natrium- und Kaliumhydrid, Lithiumdiisopropylamid sowie Lithium-, Natrium-, Kalium-, Calcium- und Bariumcarbonat. Insbesondere bevorzugte starke Basen sind die Hydroxide und Carbonate der Alkali- oder Erdalkalimetalle, ganz besonders bevorzugt die Hydroxide der Alkalimetalle. Die genannten Verbindungen können in reiner Form oder in Form von Gemischen untereinander oder in Form von Gemischen mit anderen Basen eingesetzt werden. Sie können In fester oder gelöster Form, bevorzugt in Form von wässrigen Lösungen eingesetzt werden.

Bevorzugt setzt man auch im Rahmen von Verfahrensschritt i) des erfindungsgemä-βen Verfahrens als basischen Katalysator eine wässrige Lösung eines Alkali- oder Erdalkalihydroxids, insbesondere bevorzugt eine wässrige Lösung von Natriumhydroxid ein. Setzt man die gewählte Base in Form einer Lösung, bevorzugt in Form einer wässrigen Lösung ein, beträgt der bevorzugte Konzentrationsbereich dieser Lösungen etwa 5 bis etwa 50 Gew.-% (bezogen auf die fertige Lösung), besonders bevorzugt etwa 25 bis etwa 50 Gew.-%.

Auch die Verfahrensschritte ii) und iii) des im Rahmen diese Aspekts der vorregenden Erfindung beschriebenen Verfahrens zur Herstellung von 2,6-Diphenylphenol entsprechen den Verfahrensschritten b) und c) des vorstehend beschriebenen Verfahrens zur Herstellung von 4,4'-[(1-Trifluormethyl)alkyliden]-bis-(2,6-diphenylphenolen) der Formel (I). Die gemäß Schritt ii) durchzuführende Abtrennung eines Gemisches der tricyclischen Kondensationsprodukte umfassend die Verbindungen der Formeln (IIa), (IIb) und/oder (IIc) von dem in Schritt i) gebildeten Reaktionsgemisch und die gemäß Schritt iii) durchzuführende Dehydrierung der in Schritt ii) erhaltenen tricyclischen Kondensationsprodukte umfassend die Verbindungen der Formeln (IIa), (IIb) und/oder (IIc) gemäß dem eingangs beschriebenen, erfindungsgemäßen Verfahren zur Dehydrierung unter Bildung eines 2,6-Diphenylphenols der Formel (III) kann demnach wie vorstehend für die Verfahrensschritte b) bzw. c) beschrieben, einschließlich aller bevorzugten Ausführungsformen und deren Kombinationen, vorgenommen werden. Dementsprechend wird auch Verfahrensschritt iii) wie vorstehend beschrieben in Gegenwart eines auf Al₂O₃ geträgerten Pd-Katalysators durchgeführt. Dabei kann das Al₂O₃ in Form von γ-Al₂O₃ (gamma-Al₂O₃) oder auch in Form von δ-Al₂O₃ (delta-Al₂O₃) oder in Form von θ-Al₂O₃ (theta-Al₂O₃) oder in Form von δ/θ-Al₂O₃ (delta/theta-Al₂O₃) oder in Form von α-Al₂O₃ (alpha-Al₂O₃) eingesetzt werden. Bevorzugt setzt man als Träger γ-Al₂O₃ (gamma-Al₂O₃) ein. Als im Rahmen der vorliegenden Erfindung besonders bevorzugter geträgerter Katalysator sei daher Pd auf γ-Al₂O₃ (gamma-Al₂O₃) genannt.

Palladium liegt im geträgerten Katalysator üblicherweise in einem Gewichtsanteil von etwa 0,1 bis etwa 20 Gew.-%, bevorzugt etwa 0,1 bis 10 Gew.-% (jeweils bezogen auf den fertigen Katalysator) vor. Es wird üblicherweise, je nach Art des eingesetzten Katalysators, in einer auf das Gewicht des zu dehydrierenden Gemisches tricyclischer Ketone bezogenen Menge von 1 bis 40 Gew.-%, bevorzugt 1 bis 35 Gew.-% eingesetzt.

Die Dehydrierung gemäß Verfahrensschritt iii) wird zusätzlich zum eingesetzten geträgerten Übergangsmetallkatalysator in Gegenwart von Carbonaten von Alkalimetallen durchgeführt, beispielsweise in Gegenwart von Lithium-, Natrium- oder Kaliumcarbonat. Die genannten basischen Verbindungen können, je nach Art der eingesetzten Verbindung bzw. Verbindungen in eine Menge von üblicherweise 3 bis 20 Gew.-% bezogen auf den eingesetzten geträgerten Katalysator eingesetzt werden. Alternativ lassen sich auch mit wie vorstehend genannten Alkalicarbonaten vorbehandelte Träger bzw. Trägerkatalysatoren einsetzen.

Dabei kann der jeweils wieder gewonnene Katalysator im Prinzip so lange und sooft wieder eingesetzt werden, bis er nicht mehr die gewünschte Aktivität aufweist. Dies hängt in der Regel vom jeweils gewählten Katalysator, von den gewählten Ausgangsstoffen sowie von den Reaktionsbedingungen ab. Bei Einsatz von Palladium (Pd) auf einem Träger kann dieser üblicherweise bis zu etwa zehn mal oder öfter, zumindest jedoch bis zu fünf oder bis zu vier mal zurückgeführt, d.h. wieder eingesetzt werden, ohne dass dabei nennenswerte Aktivitäts- oder Selektivitätsverluste bei der Dehydrierungsreaktion auftreten.

Der wie vorstehend beschriebene, erfindungsgemäße Zusatz von Carbonaten von Alkalimetallen im Rahmen von Verfahrensschritt iii) kann sich auch vorteilhaft auf die Aktivität, Haltbarkeit bzw. Wiederverwendbarkeit des jeweils eingesetzten geträgerten Übergangsmetallkatalysators auswirken. Der Zusatz von Alkalicarbonaten, bevorzugt von Natrium- und/oder Kaliumcarbonat und ganz besonders bevorzugt von Kaliumcarbonat (K₂CO₃) kann zu einer Aktivitätssteigerung und somit zu einer verbesserten Wiederverwendbarkeit des jeweils eingesetzten geträgerten Katalysators führen. Bei Umsetzungen mit geträgerten Palladiumkatalysatoren, speziell bei Umsetzungen mit dem erfindungsgemäß besonders bevorzugten Pd auf γ-Al₂O₃ (gamma-Al₂O₃) als Katalysator kann dieser Effekt zum Tragen kommen. Im Rahmen einer besonders bevorzugten Ausführungsform führt man demgemäß Schritt iii) des erfindungsgemäßen Verfahrens in Gegenwart von Pd auf y-Al₂O₃ (gamma-Al₂O₃) als geträgertem Übergangsmetallkatalysator und in Gegenwart eines Alkalicarbonats, bevorzugt in Gegenwart von Kaliumcarbonat durch.

Gewünschtenfalls kann im Anschluss an Verfahrensschritt iii) noch ein zusätzlicher Verfahrensschritt iv) durchgeführt werden, betreffend die Abtrennung von 2,6-Diphenylphenol der Formel (III) von dem in Schritt iii) gebildeten Reaktionsgemisch. Dieser zusätzliche Verfahrensschritt iv) entspricht dem Verfahrensschritt d) des vorstehend beschriebenen Verfahrens zur Herstellung der Verbindungen der Formel (I) und kann demnach wie vorstehend für den Verfahrensschritt d) beschrieben, einschließlich aller bevorzugten Ausführungsformen und deren Kombinationen, vorgenommen werden.

Die folgenden Beispiele dienen der Erläuterung der Erfindung, ohne sie in irgendeiner Weise zu beschränken:
Gaschromatographische Analysen wurden nach der folgenden Methode durchgeführt:
   30 m RTX 200, ID. 0.25 mm, FD: 0.50 µm; 200 °C, 3 °C/min - 290 °C; t_{R} (min) t_{R} (bicyclische Ketone der Formeln (Va, Vb)): 8.4, 8.8; t_{R} (tricyclische Ketone der Formeln (IIa, IIb, IIc)): 17.1, 18.2, 18.5; t_{R} (2-Cyclohexyl-6-phenylphenol): 15.2; t_{R} (2,6-Diphenylphenol): 18.7; t_{R} (α-Phenyldibenzofuran): 21.6. Konzentrationen der erhaltenen Rohprodukte (Gew.-%) wurden GC-analytisch mittels internem Standard ermittelt.

HPLC-Analysen wurden nach der folgenden Methode durchgeführt: CC250/4 Nucleodur C18 Gravity, 5 µm; C: Wasser - 0.05% H₃PO₄; D: Acetonitril 20 : 80; Ausgang: 93 bar, 25 °C; t_{R} (min) t_{R} (2,6-Diphenylphenol): 4.8; t_{R} (4,4'-[1-(Trifluormethyl)ethyliden]-bis-(2,6-diphenylphenol)): 14.5.

### Beispiel 1: Selbstkondensation von Cyclohexanon

In einem Kolben wurden 900 g (9.1 mol) Cyclohexanon in 190 g Xylol bei Raumtemperatur vorgelegt. Anschließend wurden 33 g (0.21 mol) NaOH-Lösung (25%) zugegeben. Die Reaktionslösung wurde unter Rückfluss gerührt. Innerhalb von 7 h stieg die Temperatur des Reaktionsgemisches von 120 auf 180°C, wobei 126 ml Wasser mittels eines Wasserabscheiders ausgekreist wurden. Die Reaktionslösung wurde anschließend auf Raumtemperatur abgekühlt.

Zur Aufarbeitung wurden 500 g Wasser zur Reaktionslösung zugegeben und mit 11 g H₃PO₄ (85%) neutralisiert. Die Phasen wurden bei 90°C getrennt. Anschließend wurde die organische Phase bei 90°C mit 500 g NaHCO₃-Lösung (2%) gewaschen. Die Phasentrennung erfolgte ebenfalls bei 90°C.

Man erhielt 965 g eines Rohproduktes folgender Zusammensetzung: Tricyclische Ketone (Formeln (IIa, IIb, IIc)): 48.3%; Bicyclische Ketone (Formeln (Va, Vb)): 24.6% (jeweils in GC-Gew.%).

Das Rohprodukt (965 g) wurde in einer mit 1 m Sulzer DX-Packung ausgerüsteten Glas-Laborkolonne (theoretische Stufenzahl von etwa 20) mit einem Innendurchmesser von 50 mm, die mit einer Blase und einem umgepumpten Dünnschichtverdampfer (0.1 m²) ausgerüstet war, batchweise destilliert. Die bicyclischen Ketone (Formeln (Va, Vb)) wurden bei 20 mbar und Kopftemperatur 144°C von den tricyclischen Ketonen (Formeln (IIa, IIb, IIc)) (Kopftemperatur 212°C; 20 mbar) abdestilliert. Man erhielt eine Ausbeute von bicyclischen Ketonen der Formeln (Va, Vb) von 223 g (27%) und von tricyclischen Ketonen der Formeln (IIa,IIb, IIc) von 410 g (50% d. Th.; 96 GC-Gew.%).

### Beispiel 2: Rückführung von bicyclischen Ketonen der Formeln (Va, Vb)

In einem Kolben wurden 360 g (3.0 mol) Cyclohexanon und 300 g (1.67 mol) bicyclische Ketone der Formeln (Va, Vb) in 140 g Xylol bei Raumtemperatur vorgelegt. Anschließend wurden 22.4 g (0.14 mol) NaOH-Lösung (25%) zugegeben. Die Reaktionslösung wurde unter Rückfluss gerührt. Innerhalb von 5 h stieg die Temperatur 120 auf 180°C, wobei 62 ml Wasser mittels eines Wasserabscheiders ausgekreist wurden. Die rote Reaktionslösung wurde auf Raumtemperatur abgekühlt und eine wie in Beispiel 1 beschriebene Aufarbeitung durchgeführt.

Man erhielt 728 g eines Rohproduktes folgender Zusammensetzung: Tricyclische Ketone (Formeln (IIa, IIb, IIc): 45.7%; Bicyclische Ketone (Formeln (Va, Vb)): 27.0%; Xylol 14.5%; Cyclohexanon: 3% (jeweils in GC-Gew.-%).

### Vergleichsbeispeile 3 bis 4 und Beispiel 5: Dehydrierung der tricyclischen Ketone der Formeln (IIa, IIb, IIc) zu 2,6-Diphenylphenol der Formel (III)

### Vergleichsbeispiel 3:

In einem Kolben wurden 10 g Katalysator Pd/Al₂O₃ (0.5 Gew.-% Palladium auf einem θ-Al₂O₃ (theta-Al₂O₃)- Träger in Form von Kugeln von 3 mm Durchmesser) in 30 g (0.11 mol) der tricyclischen Ketone der Formeln (IIa, IIb, IIc) (97%) und 0.3 g NaOH bei Raumtemperatur vorgelegt. Die Suspension wurde 4 h bei 290 bis 300°C gerührt. Nach Abkühlung des Reaktionsgemisches auf Raumtemperatur wurde das Reaktionsgemisch mit 200 ml Heptan versetzt. Die Reaktionslösung wurde auf 90°C erwärmt und anschließend der Katalysator abfiltriert und mit 100 ml Heptan nachgewaschen. Das Rohprodukt wurde am Rotationsverdampfer eingeengt.

Man erhielt ein Rohprodukt folgender Zusammensetzung: 2,6-Diphenylphenol: 71.3%, tricyclische Ketone der Formel (IIa, IIb, IIc): 8.2% (jeweils GC-Gew.-%) und 2-Cyclohexyl-6-phenylphenol: 5.0 GC-FI.-%. Das Produkt 2,6-Diphenylphenol wurde durch Kristallisation aus Heptan (120 ml) in einer Ausbeute von 62% (17.8 g, 97 GC-Gew.-%) isoliert.

### Vergleichsbeispiel 4:

In einem Kolben wurden 10 g Pd/Al₂O₃ (0.72 Gew.-% Palladium auf einem y-Al₂O₃-(gamma-Al₂O₃)Träger in Form von Strängen mit einer Länge von 4 mm) in 30 g (0.11 mol) der tricyclischen Ketone der Formeln (IIa, IIb, IIc) (97%) und 0.3 g NaOH bei Raumtemperatur vorgelegt. Die Suspension wurde 8 h bei 290 bis 300°C gerührt. Das Reaktionsgemisch wurde auf 95°C abgekühlt mit 200 ml Heptan versetzt und eine wie in Beispiel 3 beschriebene Aufarbeitung durchgeführt.

Man erhielt ein Rohprodukt folgender Zusammensetzung: 2,6-Diphenylphenol: 44.8%. tricyclische Ketone der Formel (IIa, IIb, IIc): 4.9% (jeweils GC-Gew.%) und 2-Cyclohexyl-6-phenylphenol: 12.3 GC-FI.-%.

Das Produkt 2,6-Diphenylphenol wurde durch Kristallisation aus Heptan in einer Ausbeute von 34% (9.5 g, 99 GG-FI.-%) isoliert.

### Beispiel 5:

In einem Kolben wurden 10 g Pd/Al₂O₃ (0.72 Gew.-% Palladium auf einem y-Al₂O₃-(gamma-Al₂O₃)Träger in Form von Strängen mit einer Länge von 4 mm) in 30 g (0.11 mol) der tricyclischen Ketone der Formeln (IIa, IIb, IIc) (97%) und 1.5 g K₂CO₃ bei Raumtemperatur vorgelegt. Die Suspension wurde 8 h bei 290 bis 300°C gerührt. Das Reaktionsgemisch wurde auf 90°C abgekühlt mit 200 ml Heptan versetzt und eine wie in Vergleich beispeil 3 beschriebene Aufarbeitung durchgeführt

Man erhielt ein Rohprodukt folgender Zusammensetzung: 2,6-Diphenylphenol: 47.3%, tricyclische Ketone der Formel (IIa, IIb, IIc): 5.8% (jeweils GC-Gew.%) und α-Phenyldibenzofuran: 13.6 GC-FI.-%. Das Produkt 2,6-Diphenylphenol wurde durch Kristallisation aus Heptan in einer Ausbeute von 44% (12.5 g, 98 GC-FI.-%) isoliert.

### Vergleichsbeispiel 6:

In einem Kolben wurden 10 g Katalysator Pd/Al₂O₃ (0.72 Gew.-% Palladium auf einem y-Al₂O₃-(gamma-Al₂O₃) Träger in Form von Strängen mit einer Länge von 4 mm) in 30 g (0,12 mol) der tricyclischen Ketone der Formeln (IIa, IIb, IIc) (97%) bei Raumtemperatur vorgelegt. Die Suspension wurde 8 h bei 290 bis 300°C gerührt.
Man erhielt ein Rohprodukt folgender Zusammensetzung: 2,6-Diphenylphenol: 25.7%, tricyclische Ketone der Formel (IIa, IIb, IIc): 10.3% (jeweils GC-Gew.-%) und 2-Cyclohexyl-6-phenylphenol: 34.6 GC-FI.-%.

### Vergleichsbeispiel 7:

In einem Kolben wurden 0.24 g Katalysator 5% Pd/C und 15 g (0.06 mol) der tricyclischen Ketone der Formeln (IIa, IIb, IIc) (97%) bei Raumtemperaturvorgelegt. Die Suspension wurde 2 h bei 290 bis 300°C gerührt. Die Suspension wurde abgekühlt und bei 25°C mit 50 ml Aceton verdünnt. Der Katalysator wurde abfiltriert und das Rohprodukt wurde am Rotationsverdampfer eingeengt. Anschließend wurde das Produkt 2,6-Diphenylphenol durch zweistufige Kristallisation des Rohproduktes (13 g) aus Heptan/iso-Propanol (25:1) in einer Ausbeute von 50% (7 g, 99 GC-FI.-%) isoliert.

### Beispiel 8:

In einem Kolben wurden 13.3 g Pd/Al₂O₃ (aus Beispiel 5) in 30 g (0.11 mol) der tricycl**i**schen Ketone der Formeln (IIa, IIb, IIc) (97%) bei Raumtemperatur vorgelegt. Die Suspension wurde 8 h bei 295°C gerührt. Das Reaktionsgemisch wurde auf 60°C abgekühlt mit 200 ml Heptan versetzt und eine wie in Beispiel 3 beschriebene Aufarbeitung durchgeführt. Der abgetrennte Katalysator wurde für die nächste Dehydrierungsreaktion wieder verwendet.

Man erhielt ein Rohprodukt (24.5 g) folgender Zusammensetzung: 2,6-Diphenylphenol: 54.3%, tricyclische Ketone der Formel (IIa, IIb, IIc): 8.2%, (jeweils GC-Gew.%) und 2-Cyclohexyl-6-phenylphenol: 6.9 GC-FI.-% und α-Phenyldibenzofuran: 6.3 GC-FI.-%.

### Beispiel 9: Synthese von 4,4'-[1-(Trifluormethyl)ethyliden]-bis-(2,6-diphenylphenol)

In einem Kolben wurden 61.5 g (0.25 mol) 2,6-Diphenylphenol und 875 g Methansulfonsäure (100%) vorgelegt. Anschließend wurden 15.4 g (0,14 mol) 1,1,1-Trifluoraceton bei 20°C zugegeben. Zur Vervollständigung der Reaktion wurde die Suspension 10 h bei 45°C gerührt. Die Suspension wurde anschließend auf 20°C abgekühlt und filtriert. Der Filterkuchen wurde mit destilliertem Wasser (je 730 g) neutral gewaschen und getrocknet. Man erhielt 71 g (97% d. Th.) 4,4'-[1-(Trifluormethyl)ethyliden]-bis-(2,6-diphenylphenol) in Form eines weißen Pulvers (HPLC-Gew.-%: 98%).

### Beispiel 10:

In einem Doppelmantelreaktor wurden 325 g (1.31 mol) 2,6-Diphenylphenol (99%) und 2400g Methansulfonsäure (100%) bei 20°C vorgelegt. Die Suspension wurde mit 81.3 g (0.73 mol) 1,1,1-Trifluoraceton versetzt und 10 h bei 50°C gerührt. Nach beendeter Reaktion wurde die Suspension mit 4200 g Toluol versetzt und die Reaktionsmischung 30 min bei 50°C gerührt. Die Phasen wurden bei 50°C getrennt und die ToluolPhase mit jeweils 1680 g Wasser, 1680 g 2%iger Natriumcarbonat-Lösung und mit 1680 g Wasser gewaschen. Das Lösungsmittel Toluol wurde abdestilliert. Man erhielt (376 g, 95% d. Th.) 4,4'-[1-(Trifluormethyl)ethyliden]-bis-(2,6-diphenylphenol) in Form eines weißen Pulvers (HPLC-Gew.-%: 97%).

## Patentansprüche

1. Verfahren zur Dehydrierung von Verbindungen der Formel (IIa), (IIb) und/oder (IIc) 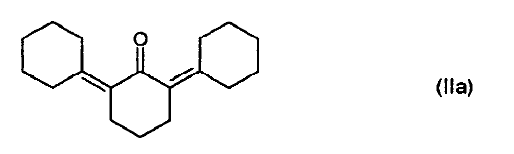 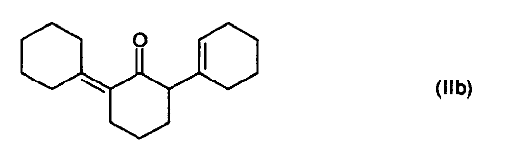 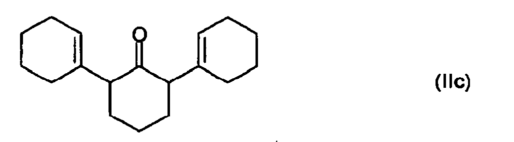 in Gegenwart eines geträgerten Übergangsmetallkatalysators in kondensierter Phase unter Bildung eines 2,6-Diphenylphenols der Formel (III) 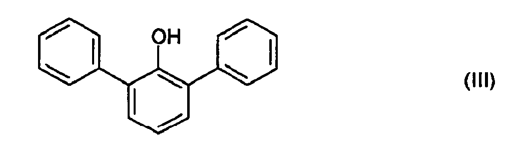 umfassenden Reaktionsgemisches, **dadurch gekennzeichnet, dass** man die Dehydrierung in Gegenwart eines auf Al₂O₃ geträgerten Pd-Katalysators und in Gegenwart von Carbonaten von Alkalimetallen durchführt.

2. Verfahren zur Herstellung von 4,4'-[1-(Trifluormethyl)alkyliden]-bis-(2,6-diphenylphenolen) der Formel (I) 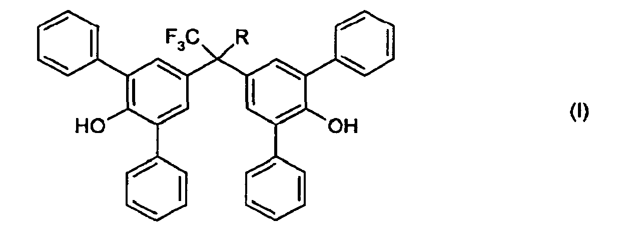 wobei der Rest
R unverzweigtes oder verzweigtes C₁- bis C₆-Alkyl oder C₁- bis C₆-Perfluoralkyl
bedeutet,
umfassend die Verfahrensschritte
a) Umsetzung von Cyclohexanon in Gegenwart eines basischen Katalysators unter Bildung eines Reaktionsgemisches umfassend die tricyclischen Kondensationsprodukte der Formel (IIa), (IIb) und/oder (IIc) gemäß Anspruch 1 und unter Bildung von Wasser,
b) Abtrennung eines Gemisches der tricyclischen Kondensationsprodukte umfassend die Verbindungen der Formeln (IIa), (IIb) und/oder (IIc) von dem in Schritt a) gebildeten Reaktionsgemisch,
c) Dehydrierung der in Schritt b) erhaltenen tricyclischen Kondensationsprodukte umfassend die Verbindungen der Formeln (IIa), (IIb) und/oder (IIc) in Gegenwart eines geträgerten Übergangsmetallkatalysators in kondensierter Phase unter Bildung eines 2,6-Diphenylphenol der Formel (III) gemäß Anspruch 1 umfassenden Reaktionsgemisches gemäß dem Verfahren zur Dehydrierung nach Anspruch 1,
d) Abtrennung von 2,6-Diphenylphenol der Formel (III) von dem in Schritt c) gebildeten Reaktionsgemisch und
e) Umsetzung des in Schritt d) erhaltenen 2,6-Diphenylphenols der Formel (III) mit einem Trifluormethylketon der Formel (IV) 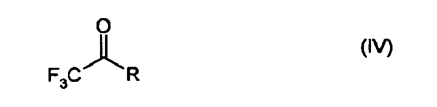 wobei der Rest R die gleiche Bedeutung wie in Formel (I) aufweist, in Gegenwart einer starken organischen Säure unter Bildung des 4,4'-[1-(Trifluomlethyl)alkyliden]-bis-(2,6-diphenylphenols) der Formel (I).

3. Verfahren nach Anspruch 2, **dadurch gekennzeichnet, dass** man in Schritt a) als basischen Katalysator eine wässrige Lösung eines Alkali- oder Erdalkalihydroxids einsetzt.

4. Verfahren nach Anspruch 2 oder 3, **dadurch gekennzeichnet, dass** man in Schritt a) eine wässrige Lösung von Natriumhydroxid als basischen Katalysator einsetzt.

5. Verfahren nach einem der der Ansprüche 2 bis 4, **dadurch gekennzeichnet, dass** man die Umsetzung gemäß Schritt a) bei einer Temperatur im Bereich von 90 bis 180°C durchführt.

6. Verfahren nach einem der Ansprüche 2 bis 5, **dadurch gekennzeichnet, dass** man die Umsetzung gemäß Verfahrensschritt a) in Gegenwart eines mit Wasser ein Azeotrop bildenden Lösungsmittels oder Lösungsmittelgemisches durchführt.

7. Verfahren nach Anspruch 6, **dadurch gekennzeichnet, dass** man das in Schritt a) gebildete Wasser in Form eines Azeotrops mit dem eingesetzten Lösungsmittel oder Lösungsmittelgemisch während der Umsetzung destillativ vom Reaktionsgemisch abtrennt.

8. Verfahren nach Anspruch 6 bis 7, **dadurch gekennzeichnet, dass** man als Lösungsmittel Xylol, Toluol oder Ethylbenzol oder Gemische derselben einsetzt.

9. Verfahren nach einem der Ansprüche 2 bis 8, **dadurch gekennzeichnet, dass** man die Abtrennung gemäß Verfahrensschritt b) in Form einer Destillation durchführt.

10. Verfahren nach einem der Ansprüche 2 bis 9, **dadurch gekennzeichnet, dass** man die Dehydrierung gemäß Verfahrensschritt c) in Gegenwart eines auf γ- Al₂O₃ geträgerten Pd-Katalysators und in Gegenwart von Kaliumcarbonat durchführt.

11. Verfahren nach einem der Ansprüche 2 bis 10, **dadurch gekennzeichnet, dass** man die Abtrennung von 2,6-Diphenylphenol gemäß Verfahrensschritt d) in Form einer Kristallisation durchführt.

12. Verfahren nach einem der Ansprüche 2 bis 11, **dadurch gekennzeichnet, dass** man die Umsetzung gemäß Verfahrensschritt e) bei einer Temperatur im Bereich von 10 bis 60°C durchführt.

13. Verfahren nach einem der Ansprüche 2 bis 12, **dadurch gekennzeichnet, dass** man die Umsetzung gemäß Verfahrensschritt e) in Gegenwart einer organischen Säure mit einem pKa-Wert von bis zu 2 durchführt.

14. Verfahren nach einem der Ansprüche 2 bis 13, **dadurch gekennzeichnet, dass** man die Umsetzung gemäß Verfahrensschritt e) in Gegenwart von Methansulfonsäure durchführt.

15. Verfahren nach einem der Ansprüche 2 bis 14, **dadurch gekennzeichnet, dass** man in Verfahrensschritt e) 2,6-Diphenylphenol und das Trifluormethylketon der Formel (IV) in einem molaren Verhältnis von 1 zu 1 bis 2 zu 1 einsetzt.

16. Verfahren nach einem der Ansprüche 2 bis 15, **dadurch gekennzeichnet, dass** der Rest R Methyl oder Trifluormethyl bedeutet.

17. Verfahren nach einem der Ansprüche 2 bis 16, **dadurch gekennzeichnet, dass** der Rest R Methyl bedeutet.

18. Verfahren nach einem der Ansprüche 2 bis 17, **dadurch gekennzeichnet, dass** man die in Verfahrensschritt e) gebildete 4,4'-[1-(Trifluormethyl)alkyliden]-bis-(2,6-diphenylphenols) der Formel (I) durch Extraktion von dem erhaltenen Reaktionsgemisch abtrennt.

19. Verfahren zur Herstellung von 2,6-Diphenylphenol der Formel **(III)** gemäß Anspruch 1
umfassend die Schritte
i) Umsetzung von Cyclohexanon in Gegenwart eines basischen Katalysators unter Bildung eines Reaktionsgemisches umfassend die tricyclischen Kondensationsprodukte der Formel (IIa), (IIb) und/oder (IIc) gemäß Anspruch 1
und unter Bildung von Wasser in Gegenwart eines mit Wasser ein Azeotrop bildenden, von Cyclohexanon verschiedenen Lösungsmittels oder Lösungsmittelgemisches, wobei man das gebildete Wasser in Form eines Azeotrops mit dem eingesetzten Lösungsmittel oder Lösungsmittelgemisch während der Umsetzung destillativ vom Reaktionsgemisch abtrennt,
ii) Abtrennung eines Gemisches der tricyclischen Kondensationsprodukte der Formel (IIa), (IIb) und/oder (IIc) von dem in Schritt i) gebildeten Reaktionsgemisch und
iii) Dehydrierung der in Schritt ii) erhaltenen tricyclischen Kondensationsprodukte umfassend die Verbindungen der Formel (IIa), (IIb) und/oder (IIc) in Gegenwart eines geträgerten Übergangsmetallkatalysators in kondensierter Phase unter Bildung eines 2,6-Diphenylphenol der Formel (III) gemäß Anspruch 1 umfassenden Reaktionsgemisches gemäß dem Verfahren zur Dehydrierung nach Anspruch 1.

## Claims

1. A process for dehydrogenating compounds of the formula (IIa), (IIb) and/or (IIc) 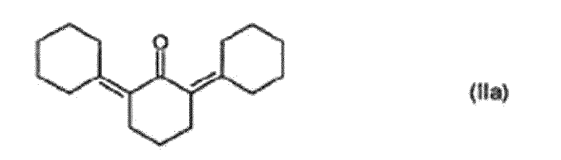 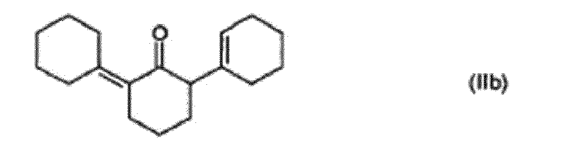 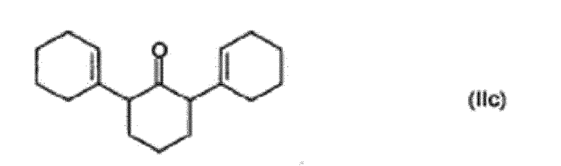 in the presence of a supported transition metal catalyst in the condensed phase to form a reaction mixture comprising 2,6-diphenylphenol of the formula (III) 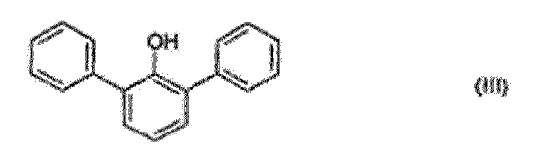 wherein the dehydrogenation is carried out in the presence of a Pd catalyst supported on Al₂O₃ and in the presence of carbonates of alkali metals.

2. A process for preparing 4,4-[1-(trifluoromethyl)alkylidene]bis(2,6-diphenylphenols) of the formula (I) 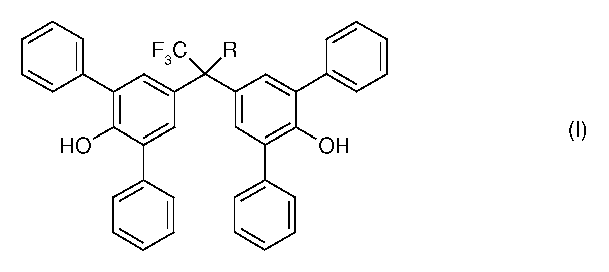 where the radical
R is unbranched or branched C₁-C₆-alkyl or C₁-C₆-perfluoroalkyl,
which comprises the process steps
a) reaction of cyclohexanone in the presence of a basic catalyst to form a reaction mixture comprising the tricyclic condensation products of the formula (IIa), (IIb) and/or (IIc) according to claim 1 and water,
b) separation of a mixture of the tricyclic condensation products comprising the compounds of the formulae (IIa), (IIb) and/or (IIc) from the reaction mixture formed in step a),
c) dehydrogenation of the tricyclic condensation products comprising the compounds of the formulae (IIa), (IIb) and/or (IIc) obtained in step b) in the presence of a supported transition metal catalyst in the condensed phase to form a reaction mixture comprising 2,6-diphenylphenol of the formula (III) according to claim 1 by the dehydrogenation process according to claim 1.
d) separation of 2,6-diphenylphenol of the formula (III) from the reaction mixture formed in step c) and
e) reaction of the 2,6-diphenylphenol of the formula (III) obtained in step d) with a trifluoromethyl ketone of the formula (IV) 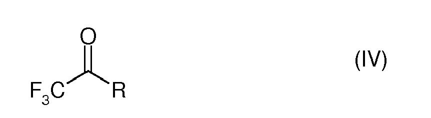
where the radical R is as defined for formula (I), in the presence of a strong organic acid to form the 4,4'-[l-(trifluoromethyl)alkylidene]bis(2,6-diphenylphenol) of the formula (I).

3. The process according to claim 2, wherein an aqueous solution of an alkali metal hydroxide or alkaline earth metal hydroxide is used as basic catalyst in step a).

4. The process according to claim 2 or 3, wherein an aqueous solution of sodium hydroxide is used as basic catalyst in step a).

5. The process according to any of claims 2 to 4, wherein the reaction according to step a) is carried out at a temperature in the range from 90 to 180°C.

6. The process according to any of claims 2 to 5, wherein the reaction according to process step a) is carried out in the presence of a solvent or solvent mixture which forms an azeotrope with water.

7. The process according to claim 6, wherein the water formed in step a) is separated from the reaction mixture by distillation in the form of an azeotrope with the solvent or solvent mixture used during the reaction.

8. The process according to claim 6 or 7, wherein xylene, toluene or ethylbenzene or a mixture thereof is used as solvent.

9. The process according to any of claims 2 to 8, wherein the separation according to process step b) is carried out in the form of a distillation.

10. The process according to any of claims 2 to 9, wherein the dehydrogenation according to process step c) is carried out in the presence of a Pd catalyst supported on γ-Al₂O₃ and in the presence of potassium carbonate.

11. The process according to any of claims 2 to 10, wherein the isolation of 2,6-diphenylphenol according to process step d) is carried out in the form of a crystallization.

12. The process according to any of claims 2 to 11, wherein the reaction according to process step e) is carried out at a temperature in the range from 10 to 60°C.

13. The process according to any of claims 2 to 12, wherein the reaction according to process step e) is carried out in the presence of an organic acid having a pKa of up to 2.

14. The process according to any of claims 2 to 13, wherein the reaction according to process step e) is carried out in the presence of methanesulfonic acid.

15. The process according to any of claims 2 to 14, wherein 2,6-diphenylphenol and the trifluoromethyl ketone of the formula (IV) are used in a molar ratio of from 1:1 to 2:1 in process step e).

16. The process according to any of claims 2 to 15, wherein the radical R is methyl or trifluoromethyl.

17. The process according to any of claims 2 to 16, wherein the radical R is methyl.

18. The process according to any of claims 2 to 17, wherein the 4,4'-[1-(trifluoromethyl)alkylidene]-bis(2,6-diphenylphenol) of the formula (I) formed in process step e) is separated off from the resulting reaction mixture by extraction.

19. A process for preparing 2,6-diphenylphenol of the formula (III) according to claim 1
which comprises the steps
i) reaction of cyclohexanone in the presence of a basic catalyst to form a reaction mixture comprising the tricyclic condensation products of the formula (IIa), (IIb) and/or (IIc) according to claim 1
and water in the presence of a solvent or solvent mixture other than cyclohexanone which forms an azeotrope with water, with the water formed being separated off from the reaction mixture by distillation in the form of an azeotrope with the solvent or solvent mixture used during the reaction,
ii) separation of a mixture of the tricyclic condensation products of the formulae (IIa), (IIb) and/or (IIc) from the reaction mixture formed in step i) and
iii) dehydrogenation of the tricyclic condensation products comprising the compounds of the formulae (IIa), (IIb) and/or (IIc) obtained in step ii) in the presence of a supported transition metal catalyst in the condensed phase to form a reaction mixture by the dehydrogenation process according to claim 1 comprising 2,6-diphenylphenol of the formula (III) according to claim 1.

## Revendications

1. Procédé pour la déshydrogénation de composés de formule (IIa), (IIb) et/ou (IIc) 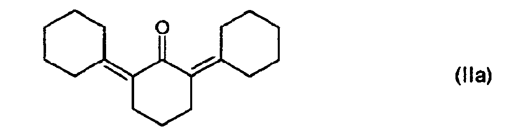 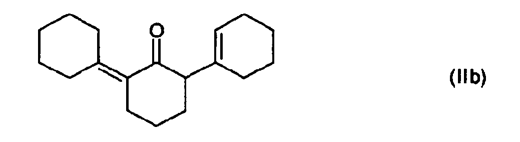 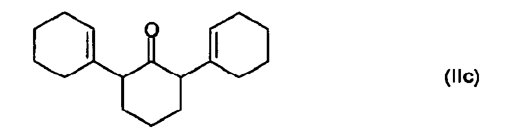 en présence d'un catalyseur de métal de transition supporté en phase condensée avec formation d'un mélange réactionnel comprenant du 2,6-diphénylphénol de formule (III) 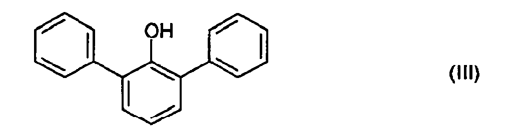 **caractérisé en ce qu'**on réalise la déshydrogénation en présence d'un catalyseur de Pd supporté sur Al₂O₃ et en présence de carbonates de métaux alcalins.

2. Procédé pour la préparation de 4,4'-[1-(trifluorométhyl)alkylidène]-bis-(2,6-diphénylphénols) de formule (I) 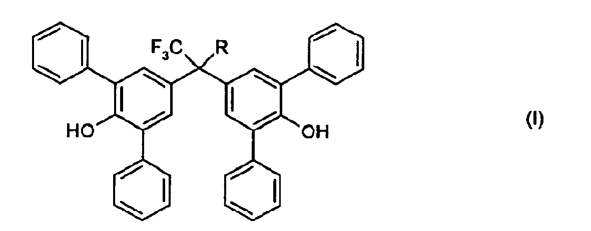 dans laquelle le radical
R signifie alkyle en C₁-C₆ ou perfluoroalkyle en C₁ à C₆ non ramifié ou ramifié,
comprenant les étapes de procédé
a) transformation de cyclohexanone en présence d'un catalyseur basique avec formation d'un mélange réactionnel comprenant les produits de condensation tricycliques de formule (IIa), (IIb) et/ou (IIc) selon la revendication 1 et avec formation d'eau,
b) séparation d'un mélange des produits de condensation tricycliques comprenant les composés des formules (IIa), (IIb) et/ou (IIc) du mélange réactionnel formé dans l'étape a),
c) déshydrogénation des produits de condensation tricycliques obtenus dans l'étape b) comprenant les composés des formules (IIa), (IIb) et/ou (IIc) en présence d'un catalyseur de métal de transition supporté en phase condensée avec formation d'un mélange réactionnel contenant du 2,6-diphénylphénol de formule (III) selon la revendication 1 selon le procédé de déshydrogénation selon la revendication 1,
d) séparation du 2,6-diphénylphénol de formule (III) du mélange réactionnel formé dans l'étape c) et
e) transformation du 2,6-diphénylphénol de formule (III) obtenu dans l'étape d) avec une trifluorométhylcétone de formule (IV) 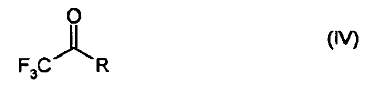 le radical R présentant la même signification que dans la formule (I) en présence d'un acide organique fort avec formation du 4,4'-[1-(trifluorométhyl)alkylidène]-bis-(2,6-diphénylphénol) de formule (I).

3. Procédé selon la revendication 2, **caractérisé en ce qu'**on utilise dans l'étape a) comme catalyseur basique une solution aqueuse d'un hydroxyde de métal alcalin ou alcalino-terreux.

4. Procédé selon la revendication 2 ou 3, **caractérisé en ce qu'**on utilise dans l'étape a) comme catalyseur basique une solution aqueuse d'hydroxyde de sodium.

5. Procédé selon l'une quelconque des revendications 2 à 4, **caractérisé en ce qu'**on réalise la transformation selon l'étape a) à une température dans la plage de 90 à 180°C.

6. Procédé selon l'une quelconque des revendications 2 à 5, **caractérisé en ce qu'**on réalise la transformation selon l'étape de procédé a) en présence d'un solvant ou d'un mélange de solvants formant un azéotrope avec l'eau.

7. Procédé selon la revendication 6, **caractérisé en ce qu'**on sépare pendant la transformation l'eau formée dans l'étape a) sous forme d'un azéotrope avec le solvant ou le mélange de solvants utilisé par distillation du mélange réactionnel.

8. Procédé selon la revendication 6 à 7, **caractérisé en ce qu'**on utilise, comme solvant, le xylène, le toluène ou l'éthylbenzène ou leurs mélanges.

9. Procédé selon l'une quelconque des revendications 2 à 8, **caractérisé en ce qu'**on réalise la séparation selon l'étape de procédé b) sous forme d'une distillation.

10. Procédé selon l'une quelconque des revendications 2 à 9, **caractérisé en ce qu'**on réalise la déshydrogénation selon l'étape de procédé c) en présence d'un catalyseur de Pd supporté sur γ-Al₂O₃ et en présence de carbonate de potassium.

11. Procédé selon l'une quelconque des revendications 2 à 10, **caractérisé en ce qu'**on réalise la séparation de 2,6-diphénylphénol selon l'étape de procédé d) sous forme d'une cristallisation.

12. Procédé selon l'une quelconque des revendications 2 à 11, **caractérisé en ce qu'**on réalise la transformation selon l'étape de procédé e) à une température dans la plage de 10 à 60°C.

13. Procédé selon l'une quelconque des revendications 2 à 12, **caractérisé en ce qu'**on réalise la transformation selon l'étape de procédé e) en présence d'un acide organique présentant une valeur de pKa de jusqu'à 2.

14. Procédé selon l'une quelconque des revendications 2 à 13, **caractérisé en ce qu'**on réalise la transformation selon l'étape de procédé e) en présence d'acide méthanesulfonique.

15. Procédé selon l'une quelconque des revendications 2 à 14, **caractérisé en ce qu'**on utilise, dans l'étape de procédé e), le 2,6-diphénylphénol et la trifluorométhylcétone de formule (IV) dans un rapport molaire de 1:1 à 2:1.

16. Procédé selon l'une quelconque des revendications 2 à 15, **caractérisé en ce que** le radical R signifie méthyle ou trifluorométhyle.

17. Procédé selon l'une quelconque des revendications 2 à 16, **caractérisé en ce que** le radical R signifie méthyle.

18. Procédé selon l'une quelconque des revendications 2 à 17, **caractérisé en ce qu'**on sépare les 4,4'-[1-(trifluorométhyl)alkylidène]-bis-(2,6-diphénylphénols) de formule (I) formé dans l'étape de procédé e) par extraction du mélange réactionnel obtenu.

19. Procédé pour la fabrication de 2,6-diphénylphénol de formule (III) selon la revendication 1, comprenant les étapes
i) transformation de cyclohexanone en présence d'un catalyseur basique avec formation d'un mélange réactionnel comprenant les produits de condensation tricycliques de formule (IIa), (IIb) et/ou (IIc) selon la revendication 1 et avec formation d'eau en présence d'un solvant ou d'un mélange de solvants différent(s) de la cyclohexanone, formant un azéotrope avec l'eau, l'eau formée étant séparée pendant la transformation sous forme d'un azéotrope avec le solvant ou le mélange de solvants utilisé par distillation du mélange réactionnel,
ii) séparation d'un mélange des produits de condensation tricycliques des formules (IIa), (IIb) et/ou (IIc) du mélange réactionnel formé dans l'étape i) et
iii) déshydrogénation des produits de condensation tricycliques obtenus dans l'étape ii) comprenant les composés des formules (IIa), (IIb) et/ou (IIc) en présence d'un catalyseur de métal de transition supporté en phase condensée avec formation d'un mélange réactionnel contenant un 2,6-diphénylphénol de formule (III) selon la revendication 1 selon le procédé de déshydrogénation selon la revendication 1.
